(19) Europäisches Patentamt
European Patent Office
Office européen des brevets

(11) Veröffentlichungsnummer: **0 608 858 A1**

(12) # EUROPÄISCHE PATENTANMELDUNG

(21) Anmeldenummer: **94101125.6**

(22) Anmeldetag: **26.01.94**

(51) Int. Cl.[5]: **C07D 417/10**, C07D 403/04, A61K 31/445, A61K 31/41, C07D 401/10, C07D 417/06, C07D 401/06, C07D 403/12, C07D 417/12, C07D 417/14, C07D 417/04, C07D 413/10

(30) Priorität: **26.01.93 DE 4302051**

(43) Veröffentlichungstag der Anmeldung: **03.08.94 Patentblatt 94/31**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach(DE)**

(72) Erfinder: **Linz, Günter, Dr.**
**Erlenweg 8**
**D-88441 Mittelbiberach(DE)**
Erfinder: **Himmelsbach, Frank, Dr.**
**Ahornweg 16**
**D-88441 Mittelbiberach(DE)**
Erfinder: **Austel, Volkhard, Prof. Dr.**
**Kapellenweg 7**
**D-88400 Biberach(DE)**
Erfinder: **Pieper, Helmut, Dr.**
**Kapellenweg 5**
**D-88400 Biberach(DE)**
Erfinder: **Müller, Thomas Dr.**
**Alter Postplatz 17**
**D-88400 Biberach(DE)**
Erfinder: **Weisenberger, Johannes, Dr.**
**Haydnweg 5**
**D-88400 Biberach(DE)**
Erfinder: **Guth, Brian, Dr.**
**Am Schlegelberg 24**
**D-88447 Warthausen(DE)**

(54) **5-Gliedrige Heterocyclen als Aggregationshemmer.**

(57) Die Erfindung betrifft 5-gliedrige Heterocyclen der allgemeinen Formel

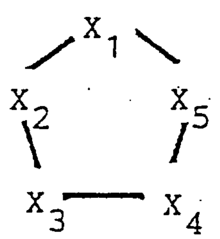

, (I)

in der
$X_1$ bis $X_5$ wie im Anspruch 1 definiert sind, deren Tautomere, deren Stereoisomere einschließlich deren Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

EP 0 608 858 A1

Die Erfindung betrifft 5-gliedrige Heterocyclen der allgemeinen Formel

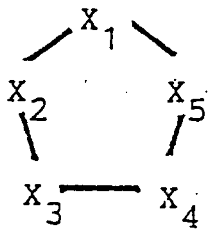

, (I)

deren Tautomere, deren Stereoisomere einschließlich deren Gemische und deren Salze, insbesondere deren physiologisch verträgliche Salze mit anorganischen oder organischen Säuren oder Basen, welche u.a. wertvolle pharmakologische Eigenschaften aufweisen, vorzugsweise aggregationshemmende Wirkungen, diese Verbindungen enthaltende Arzneimittel und Verfahren zu ihrer Herstellung.

In der obigen allgemeinen Formel I bedeutet

(i) mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält und

(ii) mit Ausnahme der Verbindungen 3-(4-Amidino-phenyl)-1-[4-(2-amino-2-carboxy-ethyl)-phenyl]-2H-pyrazol-5-on und 3-(4-Amidino-phenyl)-1-[4-(2-amino-2-methoxycarbonyl-ethyl)-phenyl]-2H-pyrazol-5-on,

einer der Reste $X_1$ bis $X_5$ eine Gruppe der Formeln

A - B - C - N< ,

A - B - C - CH< oder

A - B - C - C⩽ , in denen

A eine gegebenenfalls durch 1 bis 4 Alkylgruppen, durch eine Hydroxy- Alkoxy-, Phenylalkoxy-, Cyano-, Aminocarbonyl-, Carboxy- Alkoxycarbonyl- oder Phenylalkoxycarbonylgruppe substituierte Cycloalkylgruppe mit 5 bis 7 Kohlenstoffatomen, in der eine unsubstituierte Methylengruppe durch die $R_a$-N< Gruppe ersetzt ist, wobei

$R_a$ ein Wasserstoffatom, eine Alkylgruppe, eine Phenylalkylgruppe, eine Alkoxycarbonylgruppe mit insgesamt 2 bis 6 Kohlenstoffatomen, eine Phenylalkoxycarbonylgruppe, eine Alkenyloxycarbonylgruppe mit insgesamt 4 bis 6 Kohlenstoffatomen, eine Cycloalkoxycarbonylgruppe mit insgesamt 6 bis 8 Kohlenstoffatomen oder eine $R_1$-CO-O-($R_2$CH)-O-CO-Gruppe, in der

$R_1$ eine Alkylgruppe mit 1 bis 5 Kohlenstoffatomen, eine Cycloalkylgruppe mit 5 bis 7 Kohlenstoffatomen, eine Phenylalkylgruppe, eine Alkoxygruppe mit 1 bis 5 Kohlenstoffatomen, eine Cycloalkoxygruppe mit 5 bis 7 Kohlenstoffatomen oder eine Phenylgruppe und

$R_2$ ein Wasserstoffatom, eine Alkylgruppe mit 1 bis 4 Kohlenstoffatomen, eine Cycloalkylgruppe mit 5 bis 7 Kohlenstoffatomen oder eine Phenylgruppe darstellen,

und zusätzlich in den so gebildeten 6- oder 7-gliedrigen Azacycloalkylgruppen eine >CH- Einheit in 4-Stellung durch ein Stickstoffatom oder in den so gebildeten 5- bis 7-gliedrigen Azacycloalkylgruppen eine $-CH_2$-CH< Einheit durch eine -CH=C< Einheit und in den so gebildeten Piperazinyl- oder Homopiperazinylringen eine Methylengruppe, die benachbart zu dem Stickstoffatom in 4-Stellung steht, durch ein Carbonylgruppe ersetzt sein kann,

eine Pyridyl- oder Chinuclidinylgruppe oder auch, wenn

D eine durch eine $R_3R_4$N-CO-$NR_5$- oder $R_6$-$SO_2$-$NR_3$-Gruppe substituierte Alkylen- oder Alkenylengruppe mit jeweils bis zu 8 Kohlenstoffatomen, in denen

$R_3$ bis $R_5$, die gleich oder verschieden sein können, jeweils ein Wasserstoffatom, eine Alkyl- oder Phenylalkylgruppe und

$R_6$ eine Alkylgruppe mit 1 bis 5 Kohlenstoffatomen, eine Phenylalkyl- oder Phenylgruppe darstellen,

oder E eine durch eine Hydroxy-, Alkoxy-, Alkylsulfenyl-, $R_3R_4$N-, $R_3$O-CO-, $R_6$CO-$NR_3$-, $R_6$O-CO-$NR_3$-, $R_6SO_2$-$NR_3$- oder $R_3R_4$N-CO-$NR_5$-Gruppe substituierte geradkettige oder verzweigte Alkylen- oder Alkenylengruppe mit jeweils bis zu 5 Kohlenstoffatomen, in denen $R_3$ bis $R_6$ wie vorstehend erwähnt definiert sind, darstellen,

eine Phenylgruppe, die in 4-Stellung durch eine $R_a$NH-$CH_2$- oder $R_a$NH-C(=NH)-Gruppe substituiert ist, oder eine Phenylgruppe, in die über zwei benachbarte Kohlenstoffatome eine durch eine $R_a$NH-Gruppe substituierte n-Alkylenbrücke mit 3 oder 4 Kohlenstoffatomen angefügt ist, wobei $R_a$ jeweils wie vorstehend erwähnt definiert ist,

B eine Bindung, eine Alkylengruppe, eine $-OCH_2$-, $-CH_2$O-, $-SCH_2$-, $-CH_2$S-, $-CONR_3$-, $-R_3$NCO-, $-CH_2NR_3$-, $-NR_3CH_2$-, $-SO_2NR_3$- oder $-NR_3SO_2$-Gruppe, wobei $R_3$ wie vorstehend erwähnt definiert ist und ein

Sauerstoff- oder Stickstoffatom des Restes B nicht direkt mit einem Stickstoffatom des Restes A oder des 5-gliedrigen Heterocyclus verbunden ist,
C eine Phenylengruppe, die durch Fluor-, Chlor- oder Bromatome, durch Alkyl-, Trifluormethyl-, Hydroxy-, Alkoxy-, Alkylsulfenyl-, Alkylsulfinyl- oder Alkylsulfonylgruppen mono- oder disubstituiert sein kann, wobei die Substituenten gleich oder verschieden sein können,
eine Pyridinylen-, Pyrimidinylen-, Pyrazinylen- oder Pyridazinylengruppe, die jeweils im Kohlenstoffgerüst durch ein Chloratom, durch eine Alkyl- oder Alkoxygruppe substituiert sein können,
eine 1,4-Cyclohexylen-, 1,3-Piperidinylen-, 1,4-Piperidinylen- oder 1,4-Piperazinylengruppe oder
auch, sofern nicht A eine Pyridylgruppe und gleichzeitig B eine Alkylen-, -CONH- oder $-CH_2S$-Gruppe darstellt, eine Bindung, wobei gleichzeitig B nur dann eine Bindung sein kann, wenn A eine Amidinophenylgruppe darstellt,
ein zweiter der Reste $X_1$ bis $X_5$ eine Gruppe der Formeln

$R_bO$-CO - E - D - N< ,

$R_bO$-CO - E - D - CH< oder

$R_bO$-CO - E - D - C⋞ , in denen

D eine $-CO-NR_3-$, $-NR_3-CO-$, $-SO_2-NR_3-$ oder $-NR_3-SO_2$-Gruppe, eine gegebenenfalls durch eine Hydroxy-, Alkoxy-, Alkylsulfenyl-, $R_3R_4N-$, $R_3O-CO-$, $R_6CO-NR_3-$, $R_6O-CO-NR_3-$, $R_6SO_2-NR_3-$ oder $R_3R_4N-CO-NR_5$-Gruppe substituierte geradkettige oder verzweigte Alkylen- oder Alkenylengruppe, in denen jeweils der Alkylenteil 1 bis 8 Kohlenstoffatome und der Alkenylenteil 2 bis 8 Kohlenstoffatome enthalten kann sowie $R_3$ bis $R_6$ wie vorstehend erwähnt definiert sind,
eine Phenylengruppe, die durch Fluor-, Chlor- oder Bromatome, durch Alkyl-, Trifluormethyl-, Hydroxy-, Alkoxy-, Alkylsulfenyl-, Alkylsulfinyl- oder Alkylsulfonylgruppen mono- oder disubstituiert sein kann,
eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine $-CO-NR_3$-Gruppe, in der $R_3$ wie vorstehend erwähnt definiert ist, ersetzt sein können und eines der Stickstoffatome statt an den Rest $R_3$ auch an den Rest E, sofern dieser keine Bindung darstellt und nicht über ein Sauerstoff- oder Schwefelatom an den Rest D gebunden ist, oder an das im Ring befindliche Atom des jeweiligen Restes $X_1$ bis $X_5$ gebunden sein kann,
eine gegebenenfalls durch eine Alkyl-, Phenylalkyl- oder Phenylgruppe substituierte Cycloalkylengruppe mit 4 bis 5 Kohlenstoffatomen, in der eine >CH-Einheit durch ein Stickstoffatom und zusätzlich eine zum Stickstoffatom benachbarte Methylengruppe durch eine Carbonylgruppe ersetzt sein kann,
eine gegebenenfalls durch eine Alkyl-, Phenylalkyl- oder Phenylgruppe substituierte Cycloalkylengruppe mit 6 oder 7 Kohlenstoffatomen, in der eine oder zwei >CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, oder
eine über den Rest $W_1$ mit dem im Ring befindlichen Atom des jeweiligen Restes $X_1$ bis $X_5$ verknüpfte Alkylengruppe mit 1 bis 5 Kohlenstoffatomen, in der $W_1$ eine $NR_3$-Gruppe, in der $R_3$ wie vorstehend erwähnt definiert ist, ein Sauerstoff- oder Schwefelatom darstellt, wobei ein Sauerstoff- oder Schwefelatom des Restes $W_1$ nicht direkt an ein Stickstoffatom des 5-gliedrigen Heterocyclus gebunden sein kann,
E eine Bindung, wobei nicht gleichzeitig C eine Bindung und A eine 1-Methyl- oder 1-Benzyl-4-piperazinylgruppe darstellen kann, oder
eine gegebenenfalls durch eine Hydroxy-, Alkoxy-, Alkylsulfenyl-, $R_3R_4N-$, $R_3O-CO-$, $R_6CO-NR_3-$, $R_6O-CO-NR_3-$, $R_6SO_2-NR_3-$ oder $R_3R_4N-CO-NR_5$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann sowie $R_3$ bis $R_6$ wie vorstehend erwähnt definiert sind, oder
eine über den Rest $W_2$ mit dem Rest D verknüpfte Alkylengruppe, in der $W_2$ ein Sauerstoff- oder Schwefelatom, eine Sulfinyl-, Sulfonyl-, $-NR_3-$, $-(R_6CO)N-$, $-(R_6SO_2)N-$, $-CONR_3-$ oder $-NR_3CO$-Gruppe darstellt, in denen $R_3$ und $R_6$ wie vorstehend definiert sind und wobei ein Sauerstoff- oder Schwefelatom des Restes $W_2$ nicht direkt an ein Stickstoffatom des Restes D gebunden ist, und
$R_b$ ein Wisserstoffatom, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil oder eine $R_1-CO-O-(R_2CH)$-Gruppe, in der $R_1$ und $R_2$ wie vorstehend erwähnt definiert sind, wobei der Abstand zwischen dem am weitesten entfernten Sticktoffatom der Gruppe A und der $COOR_b$-Gruppe mindestens 11 Bindungen beträgt,
ein dritter der Reste $X_1$ bis $X_5$ ein Schwefelatom, eine HN<, $R_6N$<, $R_7C$⋞ oder $(R_7)_2C$< Gruppe oder ein N-Atom, wobei $R_6$ wie eingangs definiert ist und $R_7$ ein Wasserstoffatom, eine Alkyl-,Phenylalkyl-, Phenyl-, Alkoxy-, $R_3R_4N-$, $R_3O-CO-$ oder $R_3R_4N-CO$-Gruppe darstellt, wobei $R_3$ und $R_4$ wie vorstehend erwähnt

definiert sind,
ein vierter der Reste $X_1$ bis $X_5$ ein Sauerstoff-, Schwefel- oder Stickstoffatom oder eine $R_7C\leqslant$ Gruppe, in der $R_7$ wie vorstehend erwähnt definiert ist, oder auch eine Carbonylgruppe, wenn diese nicht zwischen zwei Stickstoffatomen steht,
ein fünfter der Reste $X_1$ bis $X_5$ ein Stickstoffatom, eine $R_7C\leqslant$ oder $(R_7)_2C<$ Gruppe, wobei $R_7$ wie vorstehend erwähnt definiere ist,
oder auch zwei benachbarte Reste der Reste $X_1$ bis $X_5$ zusammen eine o-Phenylengruppe bedeuten,
wobei, soweit nichts anderes erwähnt wurde,
die vorstehend erwähnten Alkyl-, Alkylen- oder Alkoxyteile jeweils 1 bis 3 Kohlenstoffatome enthalten können.

Unter die vorstehend erwähnte allgemeine Formel I fallen somit beispielsweise die entsprechend substituierten Furan-, Tetrahydrofuran-, 2,3-Dihydro-furan-, 2,5-Dihydro-furan-, Thiophen-, 2,3-Dihydro-thiophen-, 2,5-Dihydro-thiophen-, Tetrahydrothiophen-, 1,2-Dithiolan-, 1,3-Dithiolan-, Pyrrol-, Indol-, Isoindol-, 2,3-Dihydro-indol-, 2,3-Dihydro-isoindol-, 2-Indolon-, Imidazol-, 4,5-Dihydro-imidazol-, Tetrahydroimidazol-, Benzimidazolin-, Pyrazol-, 2H-Pyrazol-5-on-, 4,5-Dihydro-pyrazol-, 2,3-Dihydro-pyrazol-, Indazol-, 2,3-Dihydroindazol-, Oxazol-, Isoxazol-, Oxazolin-, Oxazolidin-, Thiazol-, Isothiazol-, Thiazolin-, Thiazolidin-, 1,3,4-Oxadiazol-, 1,3,4-Thiadiazol-, 1,2,3-Triazol-, 1,2,4-Triazol- und Tetrazolderivate.

Bevorzugte Verbindungen der obigen allgemeinen Formel I sind diejenigen, in denen
einer der Reste $X_1$ bis $X_5$ eine Gruppe der Formeln

A - B - C - N< ,
A - B - C - CH< oder
A - B - C - $C\leqslant$ , in denen

A eine gegebenenfalls durch 1 bis 4 Alkylgruppen, durch eine Hydroxy-, Alkoxy-, Cyano-, Aminocarbonyl-, Carboxy- oder Alkoxycarbonylgruppe substituierte Cycloalkylgruppe mit 5 bis 7 Kohlenstoffatomen, in der eine unsubstituierte Methylengruppe durch die $R_a$-N< Gruppe ersetzt ist, wobei

$R_a$ ein Wasserstoffatom, eine Alkylgruppe, eine Phenylalkylgruppe, eine Alkoxycarbonylgruppe mit insgesamt 2 bis 6 Kohlenstoffatomen, eine Phenylalkoxycarbonylgruppe, eine Cycloalkoxycarbonylgruppe mit insgesamt 6 bis 8 Kohlenstoffatomen oder eine $R_1$-CO-O-($R_2$CH)-O-CO-Gruppe, in der

$R_1$ eine Alkylgruppe, eine Cycloalkylgruppe mit 5 bis 7 Kohlenstoffatomen, eine Alkoxygruppe mit 1 bis 5 Kohlenstoffatomen, eine Cycloalkoxygruppe mit 5 bis 7 Kohlenstoffatomen oder eine Phenylgruppe und

$R_2$ ein Wasserstoffatom, eine Alkylgruppe mit 1 bis 4 Kohlenstoffatomen darstellen,

und zusätzlich in den so gebildeten 6- oder 7-gliedrigen Azacycloalkylgruppen eine >CH- Einheit in 4-Stellung durch ein Stickstoffatom oder in den so gebildeten 5- bis 7-gliedrigen Azacyclcoalkylgruppen eine $-CH_2$-CH< Einheit durch eine -CH = C< Einheit ersetzt sein kann,
eine Pyridyl- oder Chinuclidinylgruppe oder auch, wenn

D eine durch eine $R_3R_4$N-CO-$NR_5$- oder $R_6$-$SO_2$-$NR_3$-Gruppe substituierte Alkylengruppe mit 1 bis 5 Kohlenstoffatomen, in denen

$R_3$ bis $R_5$, die gleich oder verschieden sein können, jeweils ein Wasserstoffatom, eine Alkyl- oder Phenylalkylgruppe und

$R_6$ eine Alkylgruppe mit 1 bis 5 Kohlenstoffatomen, eine Phenylalkyl- oder Phenylgruppe darstellen,

oder E eine durch eine $R_3R_4$N-, $R_6$CO-$NR_3$-, $R_6SO_2$-$NR_3$-, $R_3R_4$N-CO-$NR_5$-, Hydroxy- oder Alkoxygruppe substituierte geradkettige oder verzweigte Alkylengruppe mit jeweils 1 bis 5 Kohlenstoffatomen, in denen $R_3$ bis $R_6$ wie vorstehend erwähnt definiert sind, darstellen,
eine Phenylgruppe, die in 4-Stellung durch eine $R_a$NH-$CH_2$- oder $R_a$NH-C( = NH)-Gruppe substituiert ist, oder eine Phenylgruppe, an die über die Positionen 3 und 4 eine n-Propylen- oder n-Butylenbrücke angefügt ist, wobei die n-Propylen- und n-Butylenbrücke durch eine $R_a$NH-Gruppe substituiert ist und $R_a$ jeweils wie vorstehend erwähnt definiert ist,
B eine Bindung, eine $-CH_2$-$CH_2$-, $-OCH_2$-, $-CH_2O$-, -$CONR_3$- oder -$R_3$NCO-Gruppe, wobei $R_3$ wie vorstehend erwähnt definiert ist und ein Sauerstoff- oder Stickstoffatom des Restes B nicht direkt mit einem Stickstoffatom des Restes A oder des 5-gliedrigen Heterocyclus verbunden ist,
C eine Phenylengruppe, die durch Fluor-, Chlor- oder Bromatome, durch Alkyl-, Trifluormethyl-, Hydroxy- oder Alkoxygruppen mono- oder disubstituiert sein kann, wobei die Substituenten gleich oder verschieden sein können,
eine Pyridinylen-, Pyrimidinylen-, Pyrazinylen- oder Pyridazinylengruppe, die jeweils im Kohlenstoffgerüst durch eine Alkyl- oder Alkoxygruppe substituiert sein können,
eine 1,4-Cyclohexylen-, 1,4-Piperidinylen- oder 1,4-Piperazinylengruppe oder
auch, sofern nicht A eine Pyridylgruppe und gleichzeitig B eine Ethylen- oder CONH-Gruppe darstellt, eine Bindung, wobei gleichzeitig B nur dann eine Bindung sein kann, wenn A eine Amidinophenylgruppe

darstellt,
ein zweiter der Reste $X_1$ bis $X_5$ eine Gruppe der Formeln

$R_bO$-CO - E - D - N< ,
$R_bO$-CO - E - D - CH< oder
$R_bO$-CO - E - D - C⋞ , in denen

D eine -CO-$NR_3$- oder -$NR_3$-CO-Gruppe, eine gegebenenfalls durch eine Hydroxy-, Alkoxy-, $R_3R_4N$-, $R_6CO$-$NR_3$-, $R_6O$-CO-$NR_3$-,$R_6SO_2$-$NR_3$- oder $R_3R_4N$-CO-$NR_5$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann sowie $R_3$ bis $R_6$ wie vorstehend erwähnt definiert sind,
eine Phenylengruppe, die durch Fluor-, Chlor- oder Bromatome, durch Alkyl-, Trifluormethyl-, Hydroxy- oder Alkoxygruppen mono- oder disubstituiert sein kann,
eine Pyzidinylen-, Pyrimidinylen-, Pyrazinylen- oder Pyridazinylengruppe, die jeweils im Kohlenstoffgerüst durch eine Alkyl- oder Alkoxygruppe substituiert sein kann,
eine Cyclohexylengruppe, in der eine oder zwei >CH-Einheiten je durch ein Stickstoffatom ersetzt sein können, wobei zusätzlich eine zu einem Stickstoffatom benachbarte Methylengruppe durch eine Carbonylgruppe ersetzt sein kann, oder
eine über den Rest $W_1$ mit dem im Ring befindlichen Atom des jeweiligen Restes $X_1$ bis $X_5$ verknüpfte Alkylengruppe mit 1 bis 4 Kohlenstoffatomen, in der $W_1$ eine $NR_3$-Gruppe, in der $R_3$ wie vorstehend erwähnt definiert ist, ein Sauerstoff- oder Schwefelatom darstellt, wobei ein Sauerstoff- oder Schwefelatom des Restes $W_1$ nicht direkt an ein Stickstoffatom des 5-gliedrigen Heterocyclus gebunden sein kann,
E eine Bundung, wobei nicht gleichzeitig C eine Bindung und A eine 1-Methyl- oder 1-Benzyl-4-piperazinylgruppe darstellen kann, oder
eine gegebenenfalls durch eine Hydroxy-, Alkoxy-, $R_3R_4N$-, $R_6CO$-$NR_3$-, $R_6O$-CO-$NR_3$-, $R_6SO_2$-$NR_3$- oder $R_3R_4N$-CO-$NR_5$-Gruppe substituierte geradkettige oder verzweigte Alkylengruppe mit 1 bis 5 Kohlenstoffatomen, wobei $R_3$ bis $R_6$ wie vorstehend erwähnt definiert sind, oder
eine über den Rest $W_2$ mit dem Rest D verknüpfte Alkylengruppe, in der $W_2$ ein Sauerstoff- oder Schwefelatom, eine -$NR_3$-, -($R_6CO$)N- oder -($R_6SO_2$)N-Gruppe darstellt, in denen $R_3$ und $R_6$ wie vorstehend definiert sind und wobei ein Sauerstoff- oder Schwefelatom des Restes $W_2$ nicht direkt an ein Stickstoffatom des Restes D gebunden ist, und
$R_b$ ein Wasserstoffatom, eine Alkylgruppe mit 1 bis 5 Kohlenstoffatomen, eine Phenylalkylgruppe mit 1 bis 3 Kohlenstoffatomen im Alkylteil, eine Cycloalkylgruppe mit 5 bis 7 Kohlenstoffatomen oder eine $R_1$-CO-O-($R_2CH$)-Gruppe, in der $R_1$ und $R_2$ wie vorstehend erwähnt definiert sind, wobei der Abstand zwischen dem am weitesten entfernten Stickstoffatom der Gruppe A und der $COOR_b$-Gruppe mindestens 11 Bindungen beträgt,
ein dritter der Reste $X_1$ bis $X_5$ eine HN<, $R_6N$<, $R_7C$⋞ oder $(R_7)_2C$< Gruppe oder ein N-Atom, wobei $R_6$ wie eingangs definiert ist und $R_7$ ein Wasserstoffatom, eine Alkyl- oder Phenylgruppe darstellt,
ein vierter der Reste $X_1$ bis $X_5$ ein Sauerstoff-, Schwefel- oder Stickstoffatom oder eine $R_7C$⋞ Gruppe, in der $R_7$ wie vorstehend erwähnt definiert ist,
ein fünfter der Reste $X_1$ bis $X_5$ ein Stickstoffatom, eine $R_7C$⋞ oder $(R_7)_2C$< Gruppe, wobei $R_7$ wie vorstehend erwähnt definiert ist,
oder auch zwei benachbarte Reste der Reste $X_1$ bis $X_5$ zusammen eine o-Phenylengruppe bedeuten,
insbesondere diejenigen Verbindungen, in denen
einer der Reste $X_1$ bis $X_5$ eine Gruppe der Formeln

A - B - C - N< oder
A - B - C - C⋞ , in denen

A eine gegebenenfalls im Kohlenstoffgerüst durch 1 bis 4 Methylgruppen, durch eine Hydroxy-, Methoxy-, Cyano- oder Aminocarbonylgruppe substituierte 1,3-Pyrrolidinyl-, 1,3-Piperidyl- oder 1,4-Piperidylgruppe, wobei die vorstehend erwähnten Pyrrolidinyl- und Piperidylgruppen in 1-Stellung durch den Rest $R_a$ substituiert sind und

$R_a$ ein Wasserstoffatom, eine Methylgruppe, eine Ethylgruppe, eine Benzylgruppe oder eine Alkoxycarbonylgruppe mit insgesamt 2 bis 6 Kohlenstoffatomen darstellt,

eine 1,4-Piperazinyl- oder 3,4-Dehydro-1,4-piperidylgruppe, die jeweils in 1-Stellung durch den Rest $R_a$ substituiert sind und $R_a$ wie vorstehend erwähnt definiert ist,
eine Pyridyl- oder Chinuclidinylgruppe oder auch, wenn

D eine durch eine $R_6$-$SO_2$-$NR_3$-Gruppe substituierte Ethylengruppe, in der
$R_3$ ein Wasserstoffatom, eine Methyl- oder Ethylgruppe und
$R_6$ eine Alkylgruppe mit 1 bis 5 Kohlenstoffatomen oder eine Phenylgruppe darstellen,

oder E eine durch eine Amino-, $R_6CO-NR_3$-, $R_6SO_2-NR_3$- oder Hydroxygruppe substituierte Ethylengruppe, in denen $R_3$ und $R_6$ wie vorstehend erwähnt definiert sind, darstellen,
eine Phenylgruppe, die in 4-Stellung durch eine $R_aNH-C(=NH)$-Gruppe substituiert ist,
B eine Bindung, eine $-CH_2-CH_2-$, $-OCH_2-$, $-CH_2O-$, $-CONR_3-$, oder $-R_3NCO$-Gruppe, wobei $R_3$ wie vorstehend erwähnt definiert ist und ein Sauerstoff- oder Stickstoffatom des Restes B nicht direkt mit einem Stickstoffatom des Restes A oder des 5-gliedrigen Heterocyclus verbunden ist,
C eine Phenylengruppe, die durch ein Chloratom oder durch eine Methylgruppe substituiert sein kann,
eine Pyridinylen-, Pyrimidinylen-, Pyridazinylen- oder 1,4-Piperidinylengruppe
oder auch, sofern nicht A eine Pyridylgruppe und gleichzeitig B eine Ethylen- oder CONH-Gruppe darstellt, eine Bindung, wobei gleichzeitig B nur dann eine Bindung sein kann, wenn A eine Amidinophenylgruppe darstellt,
ein zweiter der Reste $X_1$ bis $X_5$ eine Gruppe der Formeln

$R_bO-CO - E - D - N<$ oder

$R_bO-CO - E - D - C\leqslant$ , in denen

D eine $-CO-NR_3-$ oder $-NR_3-CO$-Gruppe, wobei $R_3$ wie vorstehend erwähnt definiert ist, oder eine geradkettige oder verzweigte Alkylengruppe mit 1 bis 5 Kohlenstoffatomen,
eine durch eine $R_6SO_2-NR_3$-Gruppe substituierte Ethylengruppe,
eine Phenylengruppe, die durch ein Chloratom oder eine Methylgruppe substituiert sein kann,
eine 1,4-Cyclohexylengruppe oder
eine über eine $-NR_3$-Gruppe, wobei $R_3$ wie vorstehend erwähnt definiert ist, mit dem im Ring befindlichen Atom des jeweiligen Restes $X_1$ bis $X_5$ verknüpfte Alkylengruppe mit 1 bis 4 Kohlenstoffatomen,
E eine Bindung, wobei nicht gleichzeitig C eine Bindung und A eine 1-Methyl- oder 1-Benzyl-4-piperazinylgruppe darstellen kann, oder
eine gegebenenfalls durch eine $R_6CO-NR_3$- oder $R_6SO_2-NR_3$-Gruppe substituierte Ethylengruppe, wobei $R_3$ und $R_6$ wie vorstehend erwähnt definiert sind, oder
eine $-O-CH_2$- oder $-NR_3-CH_2$-Gruppe, wobei $R_3$ wie vorstehend erwähnt definiert ist, darstellt, und
$R_b$ ein Wasserstoffatom, eine Alkylgruppe mit 1 bis 5 Kohlenstoffitomen oder eine Cycloalkylgruppe mit 5 oder 6 Kohlenstoffatomen, wobei der Abstand zwischen dem am weitesten entfernten Stickstoffatom der Gruppe A und der $COOR_b$-Gruppe mindestens 11 Bindungen beträgt,
ein dritter der Reste $X_1$ bis $X_5$ eine HN<, $R_6N<$ oder $R_7C\leqslant$ Gruppe oder ein N-Atom, wobei $R_6$ wie vorstehend erwähnt definiert ist und $R_7$ ein Wasserstoffatom, eine Methyl- oder Ethylgruppe darstellen,
ein vierter der Reste $X_1$ bis $X_5$ ein Sauerstoff-, Schwefel- oder Stickstoffatom oder eine $R_7C\leqslant$ Gruppe, in der $R_7$ wie vorstehend erwähnt definiert ist,
ein fünfter der Reste $X_1$ bis $X_5$ ein Stickstoffatom oder eine $R_7C\leqslant$ Gruppe, wobei $R_7$ wie vorstehend erwähnt definiert ist,
oder auch zwei benachbarte Reste der Reste $X_1$ bis $X_5$ zusammen eine o-Phenylengruppe bedeuten,
deren Tautomere, deren Stereoisomere einschließlich deren Gemische und deren Salze, insbesondere deren physiologisch verträgliche Salze mit anorganischen oder organischen Säuren oder Basen.

Besonders bevorzugte Verbindungen der obigen allgemeinen Formel sind jedoch diejenigen, in denen einer der Reste $X_1$ bis $X_5$ eine Gruppe der Formeln

A - B - C - N< oder

A - B - C - $C\leqslant$ , in denen

A eine in 1-Stellung durch den Rest $R_a$ substituierte 4-Piperidylgruppe, wobei

$R_a$ ein Wasserstoffatom, eine Benzylgruppe oder eine Alkoxycarbonylgruppe mit insgesamt 2 bis 6 Kohlenstoffatomen darstellt,

eine 1,4-Piperazinylgruppe, die in 1-Stellung durch den Rest $R_a$ substituiert ist, wobei $R_a$ wie vorstehend erwähnt definiert ist
oder auch, wenn

D eine durch eine $R_6-SO_2-NR_3$-Gruppe substituierte Ethylengruppe, in der

$R_3$ ein Wasserstoffatom und

$R_6$ eine Alkylgruppe mit 1 bis 5 Kohlenstoffatomen darstellen,

eine Phenylgruppe, die in 4-Stellung durch eine $NH_2-C(=NH)$-Gruppe substituiert ist,
B eine Bindung, eine $-CH_2-CH_2-$ oder $-CH_2O$-Gruppe,
C eine Phenylen- oder eine 1,4-Piperidinylengruppe oder, falls B nicht gleichzeitig ebenfalls eine Bindung darstellt, auch eine Bindung,
ein zweiter der Reste $X_1$ bis $X_5$ eine Gruppe der Formel

$R_bO-CO - E - D - C\leqslant$ , in der

D eine -CO-NH-Gruppe, eine Alkylengruppe mit 1 bis 5 Kohlenstoffatomen, eine Phenylengruppe, eine 1,4-Cyclohexylengruppe oder eine durch eine $R_6$-$SO_2$-$NR_3$-Gruppe substituierte Ethylengruppe,
E eine Bindung, wobei nicht gleichzeitig C eine Bindung und A eine 1-Benzyl-4-piperazinylgruppe darstellen kann, oder eine Ethylengruppe und
$R_b$ ein Wasserstoffatom oder eine Alkylgruppe mit 1 bis 4 Kohlenstoffatomen, wobei der Abstand zwischen dem am weitesten entfernten Stickstoffatom der Gruppe A und der $COOR_b$-Gruppe mindestens 11 Bindungen beträgt,
ein dritter der Reste $X_1$ bis $X_5$ eine Phenyl-N< oder $R_7C\leqslant$ Gruppe oder ein N-Atom, wobei $R_7$ ein Wasserstoffatom, eine Methyl- oder Ethylgruppe darstellt,
ein vierter der Reste $X_1$ bis $X_5$ ein Sauerstoff-, Schwefel- oder Stickstoffatom oder eine $R_7C\leqslant$ Gruppe, in der $R_7$ wie vorstehend erwähnt definiert ist,
ein fünfter der Reste $X_1$ bis $X_5$ ein Stickstoffatom bedeuten,
deren Tautomere, deren Stereoisomere einschließlich deren Gemische und deren Salze, insbesondere deren physiologisch verträgliche Salze mit anorganischen oder organischen Säuren oder Basen.

Als besonders bevorzugte Verbindungen seien folgende genannt:

(i) 2-(trans-4-Carboxy-cyclohexyl)-5-[4-(4-piperidyl)-phenyl]-1,3,4-thiadiazol,
(ii) 2-[trans-4-(Methoxycarbonyl)-cyclohexyl]-5-[4-(4-piperidyl)-phenyl]-1,3,4-thiadiazol,
(iii) 1-[6-(4-Amidino-phenyl)-3-pyridazinyl]-4-[2-(n-butansulfonylamino)-2-carboxy-ethyl]-imidazol,
(iv) 1-[6-(4-Amidino-phenyl)-3-pyridazinyl]-4[2-(n-butansulfonylamino)-2-(methoxycarbonyl)-ethyl]-imidazol,
(v) 2-[trans-4-(Isobutyloxycarbonyl)-cyclohexyl]-5-[4-(4-piperidinyl)-phenyl]-1,3,4-thiadiazol und
(vi) 2-[trans-4-(Ethyloxycarbonyl)-cyclohexyl]-5-[4-(4-piperidinyl)-phenyl]-1,3,4-thiadiazol

sowie deren Salze.

Erfindungsgemäß erhält man die neuen Verbindungen beispielsweise nach folgenden Verfahren:

a) Zur Herstellung von Verbindungen der allgemeinen Formel I, in der ein zweiter der Reste $X_1$ bis $X_5$ eine $R_bO$-CO-E-D-CH<- oder $R_bC$-CO-E-D-$C\leqslant$ Gruppe darstellt:
Umsetzung einer Verbindung der allgemeinen Formel

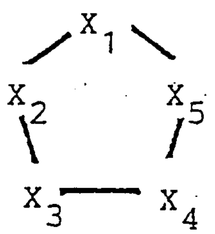

, (II)

in der
$X_1$ bis $X_5$ mit der Maßgabe wie eingangs definiert sind, daß ein zweiter der Reste $X_1$ bis $X_5$ eine HO-CO-CH< -oder HO-CO-$C\leqslant$ Gruppe darstellt, oder deren reaktionsfähigen Derivate mit einer Verbindung der allgemeinen Formel

$R_bO$ - CO - E - $HNR_3$ ,(III)

in der
$R_3$, $R_b$ und E wie eingangs definiert sind.

Die Umsetzung wird zweckmäßigerweise in einem Lösungsmittel oder Lösungsmittelgemisch wie Methylenchlorid, Dimethylformamid, Dimethylsulfoxid, Benzol, Toluol, Chlorbenzol, Tetrahydrofuran, Benzol/Tetrahydrofuran oder Dioxan gegebenenfalls in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid,2-[(1H)-Benzotriazolyl]-1,1,3,3-tetramethyl-uronium-Salzen, N,N'-Thionyldiimidazol, N,N'-Carbonyldiimidazol oder Triphenylphosphin/Tetrachlorkohlenstoff, gegebenenfalls in Gegenwart von Dimethylaminopyridin oder 1-Hydroxybenzotriazol und/oder einer Base wie Triethylamin, N-Ethyl-diisopropylamin oder N-Methyl-morpholin, zweckmäßigerweise bei Temperaturen zwischen -10 und 150 °C, vorzugsweise bei Temperaturen zwischen 0 und 50 °C, durchgeführt.

b) Zur Herstellung von Verbindungen der allgemeinen Formel I, in der $R_a$ ein Wasserstoffatom darstellt:
Abspaltung eines Schutzrestes von einer Verbindung der allgemeinen Formel

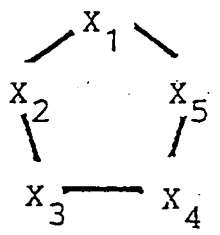

, (IV)

in der
$X_1$ bis $X_5$ mit der Maßgabe wie eingangs definiert sind, daß $R_a$ eine Alkoxycarbonylgruppe mit insgesamt 2 bis 6 Kohlenstoffatomen, eine Phenylalkoxycarbonylgruppe, eine Alkenyloxycarbonylgruppe mit insgesamt 4 bis 6 Kohlenstoffatomen, eine Cycloalkoxycarbonylgruppe mit insgesamt 6 bis 8 Kohlenstoffatomen oder einen abspaltbaren Schutzrest für eine Iminogruppe wie die Acetyl-, Trifluoracetyl-, Benzoyl-, Benzyl-, Methoxybenzyl- oder 2,4-Dimethoxybenzylgruppe darstellt, mittels Hydrolyse, Hydrogenolyse oder Thermolyse.

Die Hydrolyse wird zweckmäßigerweise entweder in Gegenwart einer Säure wie Salzsäure, Bromwasserstoffsäure, Schwefelsäure, Phosphorsäure, Essigsäure, Essigsäure/Salzsäure, Trichloressigsäure oder Trifluoressigsäure oder in Gegenwart einer Base wie Lithiumhydroxid, Natriumhydroxid oder Kaliumhydroxid in einem geeigneten Lösungsmittel wie Wasser, Methanol, Wasser/Methanol, Äthanol, Wasser/Äthanol, Wasser/Isopropanol, Wasser/Tetrahydrofuran, Ether/Dioxan oder Wasser/Dioxan bei Temperaturen zwischen -10 °C und 120 °C, z.B. bei Temperaturen zwischen Raumtemperatur und der Siedetemperatur des Reaktionsgemisches, durchgeführt.

Bei der sauren Hydrolyse können je nach den angewandten Bedingungen auch andere in einer Verbindung der Formel IV gegebenenfalls vorhandene hydrolytisch abspaltbare Gruppen wie Alkoxycarbonyl- oder Phenylalkoxycarbonylgruppen gleichzeitig abgespalten werden.

Bedeutet beispielsweise $R_a$ die tert. Butyloxycarbonylgruppe, so kann die tert. Butylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methanol, Methylenchlorid, Chloroform, Benzol, Toluol, Tetrahydrofuran, Dioxan, Ether/Dioxan oder Ether/Dioxan/Methanol vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet beispielsweise $R_a$ die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe oder eine Benzyloxygruppe zur Hydroxygruppe und eine Benzylaninogruppe zu einer Aminogruppe mitreduziert werden. Außerden können gleichzeitig vorhandene C = C-Doppelbindungen zu Einfachbindungen aufhydriert werden.

c) Zur Berstellung von Verbindungen der allgemeinen Formel I, in der $R_b$ ein Wasserstoffatom darstellt:
Abspaltung eines Schutzrestes von einer Verbindung der allgemeinen Formel

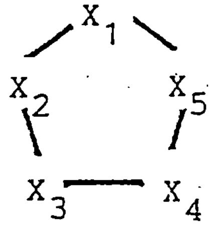

, (V)

in der
$X_1$ bis $X_5$ mit der Maßgabe wie eingangs definiert sind, daß $R_b$ Eine Alkylgruppe mit 1 bis 5

Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil oder einen abspaltbaren Schutzrest für eine Carboxygruppe wie die Trimethylsilyl-, Methoxybenzyl-, 2,4-Dimethoxybenzyl- oder Tetrahydropyranylgruppe darstellt, mittels Hydrolyse, Hydrogenolyse oder Thermolyse.

Die Hydrolyse wird zweckmäßigerweise entweder in Gegenwart einer Säure wie Salzsäure, Schwefelsäure, Phosphorsäure, Essigsäure, Essigsäure/Salzsäure, Trichloressigsäure oder Trifluoressigsäure oder in Gegenwart einer Base wie Lithiumhydroxid Natriumhydroxid oder Kaliumhydroxid in einem geeigneten Lösungsmittel wie Wasser, Methanol, Wasser/Methanol, Äthanol, Wasser/Äthanol, Wasser/Isopropanol, Wasser/Tetrahydrofuran oder Wasser/Dioxan bei Temperaturen zwischen -10°C und 120°C, z.B. bei Temperaturen zwischen Raumtemperatur und der Siedetemperatur des Reaktionsgemisches, durchgeführt.

Bei der sauren Hydrolyse können je nach den angewandten Bedingungen auch andere in einer Verbindung der Formel V gegebenenfalls vorhandene hydrolytisch abspaltbare Gruppen wie die Acetyl-, Trifluoracetyl-, Benzoyl-, tert.Butyloxycarbonyl- oder Benzyloxycarbonylgruppe gleichzeitig abgespalten werden.

Bedeutet beispielsweise $R_b$ die tert. Butylgruppe, so kann die tert Butylgruppe auch durch Behandlung mit einer Säure wie Trifluoressigsäure, Salzsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10°C und 120°C, z.B. bei Temperaturen zwischen 0 und 60°C, oder auch thermisch gegebenenfalls in einem inerten Lösungsmittel wie Methylenchlorid, Chloroform, Benzol, Toluol, Tetrahydrofuran oder Dioxan und gegebenenfalls in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet beispielsweise $R_b$ die Benzylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 10 bar abgespalzen werden. Bei der Hydrogenolyse können gleichzeitig andere Reste, z.B. eine Nitrogruppe zur Aminogruppe oder eine Benzyloxygruppe zur Hydroxygruppe und eine Benzylamino- oder Benzyloxycarbonylaminogruppe zu einer Aminogruppe mitreduziert werden. Außerdem können gleichzeitig C=C-Doppelbindung zu Einfachbindungen aufhydriert werden.

d) Zur Herstellung von 1,3,4-Oxadiazol-, 1,2,4-Triazol- und 1,3,4-Thiadiazolderivaten der allgemeinen Formel I:

Cyclisierung einer gegebenenfalls im Reaktionsgemisch gebildeten Verbindung der allgemeinen Formel

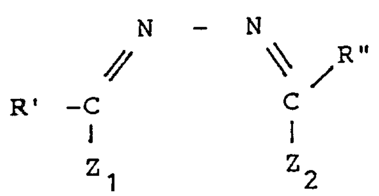

, (VI)

in der

$Z_1$ und $Z_2$, die gleich oder verschieden sein können, Halogenatome, gegebenenfalls durch $R_6$ substituierte Aminogruppen, Hydroxy-, Alkoxy-, Mercapto- oder Alkylmercaptogruppen, einer der Reste R' oder R'' eine A-B-C-Gruppe und

der andere der Reste R' oder R'' eine $R_bO$-CO-E-D-Gruppe darstellen, und erforderlichenfalls anschließende Alkylierung.

Die Umsetzung wird zweckmäßigerweise in einem Lösungsmittel wie Tetrahydrofuran, Dioxan, 1,2-Dichlorbenzol oder Pyridin bei Temperaturen bis zur Siedetemperatur des verwendeten Lösungsmittels, z.B. bei Temperaturen zwischen 20 und 180°C, durchgeführt.

Bedeutet in einer Verbindung der allgemeinen Formel VI $Z_1$ und $Z_2$ jeweils eine Hydroxygruppe, so wird zur Herstellung eines 1,3,4-Oxadiazolderivates die Umsetzung vorzugsweise in Gegenwart eines wasserentziehenden Mittels wie Thionylchlorid,

zur Herstellung eines 1,3,4-Thiadiazolderivates die Umsetzung vorzugsweise in Gegenwart eines schwefeleinführenden Reagenzwie 2,4-Bis(4-methoxyphenyl)-1,3-dithia-2,4-diphosphetan-2,4-disulfid und zur Herstellung eines 1,3,4-Tria-zolderivates die Umsetzung vorzugsweise in Gegenwart eines halogeneinführenden Mittels wie Phosphortrichlorid und in Gegenwart von Anilin durchgeführt.

Zur Herstellung der übrigen 1,2,4-Triazolderivaten wird eine Verbindung der allgemeinen Formel VI, in der einer der Reste $Z_1$ oder $Z_2$ eine Hydroxygruppe und der andere der Reste $Z_1$ oder $Z_2$ eine Aminogruppe darstellt, cyclisiert und erforderlichenfalls anschließend alkyliert.

Die anschließende Alkylierung wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

e) Zur Herstellung von Verbindungen der allgemeinen Formel I, in der A eine Phenylgruppe darstellt, die in 4-Stellung durch eine $R_a$NH-C( = NH)-Gruppe substituiert ist:
Umsetzung einer gegebenenfalls im Reaktionsgemisch gebildeten Verbindung der allgemeinen Formel

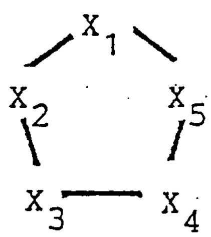

, (VII)

in der
$X_1$ bis $X_5$ mit der Maßgabe wie eingangs definiert sind, daß A eine Phenylgruppe darstellt, die in 4-Stellung durch eine $Z_3$-C( = NH)-Gruppe substituiert ist, wobei $Z_3$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_a' - NH_2$ ,(VIII)

in der
$R_a'$ ein Wasserstoffatom oder eine Alkylgruppe mit 1 bis 3 Kohlenstoffatomen darstellt.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150 °C, vorzugsweise bei Temperaturen zwischen 20 und 120 °C, mit einem entsprechenden freien Amin oder mit einem entsprechenden Säureadditionssalz wie beispielsweise Ammoniumcarbonat oder Ammoniumacetat durchgeführt.

Eine Verbindung der allgemeinen Formel VII erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50 °C, vorzugsweise jedoch bei Temperaturen zwischen 0 und 20 °C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

f) Zur Herstellung von Verbindungen der allgemeinen Formel I, in der A eine Phenylgruppe darstellt, die in 4-Stellung durch eine $R_a$NH-C( = NH)Gruppe substituiert ist:
Umsetzung einer Verbindung der allgemeinen Formel

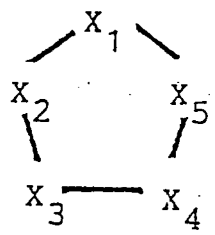

, (IX)

in der
$X_1$ bis $X_5$ mit der Maßgabe wie eingangs definiert sind, daß A eine Phenylgruppe darstellt, die in 4-Stellung durch eine Cyanogruppe substituiert ist, mit Hydroxylamin und anschließender Reduktion des so erhaltenen Amidoxims.

Die Umsetzung mit Hydroxylamin wird zweckmäßigerweise in einem Lösungsmittel wie Methanol, Ethanol, n-Propanol, Wasser, Methanol/Wasser, Ethanol/Wasser, Tetrahydrofuran oder Dioxan gegebenenfalls unter Zusatz einer Base wie z. B. Natriumcarbonat bei Temperaturen zwischen 0 und 100 °C, vorzugsweise jedoch bei Temperaturen zwischen 20 und 80 °C entweder mit freiem Hydroxylamin oder mit einem entsprechenden Säureadditionssalz wie beispielsweise dem Hydrochlorid durchgeführt.

Die nachfolgende Reduktion wird vorzugsweise in einem geeigneten Lösungsmittel wie Methanol, Methanol/Wasser, Methanol/Ammoniak, Methanol/Wasser/Ammoniak, Methanol/Salzsäure, Ethanol, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle bei Temperaturen zwischen 0 und 100 °C, vorzugsweise bei Temperaturen zwischen 20 und 80 °C, durchgeführt.

g) Zur Herstellung von Verbindungen der allgemeinen Formel I, in der A eine Phenylgruppe darstellt, die in 4-Stellung durch eine $R_aNH-C(=NH)$-Gruppe substituiert ist:
Umsetzung einer Verbindung der allgemeinen Formel

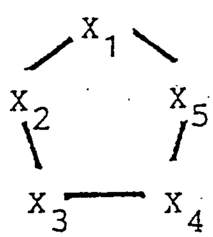

, (IX)

in der
$X_1$ bis $X_5$ mit der Maßgabe wie eingangs definiert sind, daß A eine Phenylgruppe darstellt, die in 4-Stellung durch eine Cyanogruppe substituiert ist, mit einem entsprechenden Alkylchloroaluminiumamid.

Die Umsetzung wird vorzugsweise in einem geeigneten Lösungsmittel, beispielsweise in Benzol oder Toluol, bei Temperaturen zwischen 0 und 100 °C, vorzugsweise jedoch bei einer Temperatur zwischen 20 und 80 °C durchgeführt und der so erhaltene Aluminiumkomplex anschließend hydrolytisch zersetzt, vorzugsweise mit Hilfe einer Aufschlämmung von Kieselgel in Chloroform (siehe R. S. Garigipati, Tetrahedron Letters 31, 1969 (1990)).

h) Zur Herstellung von 1,3-Thiazolen und Imidazolen der allgemeinen Formel I:
Umsetzung einer Verbindung der allgemeinen Formel

$$R' - CO - CH_2 - Z_4 \quad ,(X)$$

mit einer Verbindung der allgemeinen Formel

$$R'' - CU - NH_2 \quad ,(XI)$$

in denen
einer der Reste R' oder R'' eine A-B-C-Gruppe und der andere der Reste R' oder R'' eine $R_bO$-CO-E-D-Gruppe, $Z_4$ eine nukleophile Austrittsgruppe wie ein Chlor-, Brom- oder Jodatom und
U ein Schwefelatom oder eine Iminogruppe darstellen.

Die Umsetzung wird zweckmäßigerweise in einem Lösungsmittel wie Methanol, Ethanol oder Isopropanol gegebenenfalls in Gegenwart einer Base wie Natriumcarbonat bei erhöhten Temperaturen, z.B. bei der Siedetemperatur des verwendeten Lösungsmittels, durchgeführt.

i) Zur Herstellung von Verbindungen der allgemeinen Formel I, in der $R_a$ mit Ausnahme des Wasserstoffatoms wie eingangs definiert ist und B eine $-CH_2O$-Gruppe darstellt:

Umsetzung einer Verbindung der allgemeinen Formel

$A - CH_2 - Z_5$ ,(XII)

in der

A mit der Maßgabe wie eingangs definiert ist, daß $R_a$ mit Ausnahme des Wasserstoffatoms wie eingangs definiert ist, und $Z_5$ eine nukleophile Austrittsgruppe wie ein Halogenatom oder eine Sulfonyloxygruppe, z.B. eine Methansulfonyloxygruppe, ein Chlor- oder Bromatom, darstellt, mit einer Verbindung der allgemeinen Formel

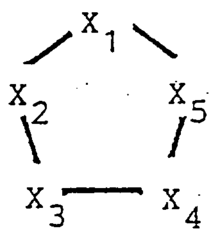

, (XIII)

in der

$X_1$ bis $X_5$ mit der Maßgabe wie eingangs definiert sind, daß einer der Reste $X_1$ bis $X_5$ eine HO-C-CH<- oder HO-C-C< Gruppe darstellt, wobei C wie eingangs definiert ist, oder mit derei Alkali- oder Erdalkalimetallsalzen wie dem Lithium-, Natrium-, Kalium-, Cäsium-, Magnesium- oder Calciumsalzen.

Die Umsetzung wird zweckmäßigerweise in einem Lösungsmittel wie Tetrahydrofuran, Aceton, Dioxan, Dimethylsulfoxid, Sulfolan, Dimethylformamid oder Dimethylacetamid in Gegenwart einer anorganischen Base wie Kaliumcarbonat, Cäsiumcarbonat, Natriumhydroxid, Kaliumhydroxid, Natriumhydrid oder Kalium-tert.butylat oder in Gegenwart tertiärer organischer Basen wie N-Ethyl-diisopropylamin, welche gegebenenfalls auch als Lösungsmittel dienen können, und gegebenenfalls in Gegenwart eines Phasentransfer-Katalysators wie Polyethylenglykol-750-monomethylether an Polystyrol oder Hexadecyltrimethylammoniumchlorid bei Temperaturen zwischen 0 und 180 ° C, vorzugsweise jedoch bei Temperaturen zwischen 10 und 160 ° C, durchgeführt.

j) Zur Herstellung von Verbindungen der allgemeinen Formel I, in der einer der Reste $X_1$ bis $X_5$ eine A-B-C-N< oder $R_b$O-CO-E-D-N< Gruppe darstellt:

Alkylierung einer Verbindung der allgemeinen Formel

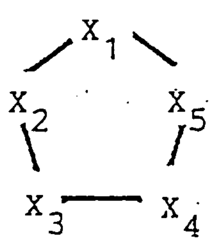

, (XIV)

in der

$X_1$ bis $X_5$ mit der Maßgabe wie eingangs definiert sind, daß eine der Reste $X_1$ bis $X_5$ eine Iminogruppe darstellt, mit einer Verbindung der allgemeinen Formel

$W - Z_6$ ,(XV)

in der

W eine A-B-C- oder $R_b$O-CO-E-D-Gruppe, wobei A bis D wie eingangs erwähnt definiert sind, und

$Z_6$ eine nukleophile Austrittsgruppe wie ein Halogenatom oder eine Sulfonyloxygruppe, z.B. eine

Methansulfonyloxygruppe, ein Chlor- oder Bromatom, darstellen.

Die Alkylierung wird zweckmäßigerweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan in Gegenwart eines säurebindendei Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

k) Zur Herstellung von Verbindungen der allgemeinen Formel I, in der $R_b$ eine $R_1$-CO-O-($R_2$CH)-Gruppe, in der $R_1$ und $R_2$ wie vorstehend erwähnt definiert sind, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil darstellt:

Veresterung einer Verbindung der allgemeinen Formel

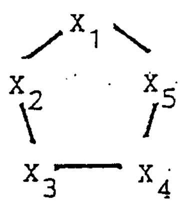

, (XVI)

in der

$X_1$ bis $X_5$ mit der Maßgabe wie eingangs definiert sind, daß $R_b$ ein Wasserstoffatom darstellt, mit einer Verbindung der allgemeinen Formel

$Z_7$ - $R_b$'     ,(XVII)

in der

$R_b$' eine $R_1$-CO-O-($R_2$CH)-Gruppe, in der $R_1$ und $R_2$ wie eingangs definiert sind, eine Alkylgruppe mit 1 bis 5 Kohlenstoffatomen, eine Alkenylgruppe mit 3 bis 5 Kohlenstoffatomen, einer Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil darstellt und

$Z_7$ eine Hydroxygruppe oder eine nukleophile Austrittsgruppe wie ein Halogenatom oder eine Sulfonyloxygruppe, z.B. ein Chlor-, Brom- oder Jodatom, eine Methansulfonyloxy- oder p-Toluolsulfonyloxygruppe, darstellen.

Die Veresterung wird zweckmäßigerweise in einem geeigneten Lösungsmittel, z. B. in einem entsprechenden Alkohol wie Methanol, Ethanol oder Isopropanol, Methylenchlorid, Tetrahydrofuran, Dioxan, Pyridin, Toluol oder Dimethylsulfoxid in Gegenwart eines säureaktivierenden und/oder wasserentziehenden Mittels wie Chlorwasserstoff, konz. Schwefelsäure, Thionylchlorid, Chlorameisensäureethylester, Carbonyldiimidazol oder N,N'-Dicyclohexyl-carbodiimid oder dessen Isoharnstoffestern, gegebenenfalls in Gegenwart eines Reaktionsbeschleunigers wie Kupferchlorid, durch Umesterung, z. B. mit einem entsprechenden Kohlensäurediester, oder durch Umsetzung mit einem entsprechenden Halogenid vorzugsweise in Gegenwart einer Base wie Kaliumcarbonat und gegebenenfalls in Gegenwart eines Reaktionsbeschleunigers wie Kaliumjodid bei Temperaturen zwischen 0 und 100°C, vorzugsweise jedoch bei Temperaturen zwischen 20°C und der Siedetemperatur des betreffenden Lösungsmittels, durchgeführt.

Bedeutet $Z_7$ eine nukleophile Austrittsgruppe, so wird die Umsetzung vorzugsweise mit einem Alkalisalz einer Verbindung der allgemeinen Formel XVI durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-,

Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 ° C, vorzugsweise bei Temperaturen zwischen 10 und 50 ° C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50 ° C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer-(IV)ammoniumnitrat in einem Lösungsmittel wie Methylenchlorid, Acetonitril oder Acetonitril/Wasser bei Temperaturen zwischen 0 und 50 ° C, vorzugsweise jedoch bei Raumtemperatur, erfolgen.
Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol Ethanol, Isopropanol, Toluol/Wasser oder Dioxan bei Temperaturen zwischen 20 und 50 ° C.
Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100 ° C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70 ° C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und chirale Verbindungen in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 stereogenen Zentren auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure,

Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren (siehe Beispiele I bis XXI),

Wie bereits eingangs erwähnt, weisen die neuen 5-gliedrigen Heterocyclen der allgemeinen Formel I und deren Salze, insbesondere deren physiologisch verträgliche Salze mit anorganischen oder organischen Säuren oder Basen, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen. Hierbei stellen die Verbindungen der allgemeinen Formel I, in der $R_a$ eine der eingangs erwähnten Oxycarbonylreste und/oder $R_b$ eine der eingangs erwähnten Esterreste darstellen, Prodrugs dar.

Beispielsweise wurden die Verbindungen der allgemeinen Formel I auf ihre biologischen Wirkungen wie folgt untersucht:

1. Kompetitive Bindung $^3$H-BIBU 52/Testsubstanz an Humanthrombozyten:

Eine Suspension von Humanthrombozyten in Plasma wird mit $^3$H-BIBU 52 [= (3S,5S)-5-[(4'-Amidino-4-biphenylyl)oxymethyl]-3-[(carboxyl)methyl]-2-pyrrolidinon[3-$^3$H-4-biphenylyl]], das den literaturbekannten Liganden $^{125}$I-Fibrinogen ersetzt, (siehe deutsche Patentanmeldung P 42 14 245.8 der gleichen Anmeldezin vom 30. 04. 1992, internes Zeichen: Case 5/1093-FL) und
verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

Hierzu wird aus einer Antikubitalvene Spenderblut entnommen und mit Trinatriumzitrat antikoaguliert (Endkonzentration 13 mM) . Das Blut wird 10 Minuten bei 170 x g zentrifugiert und das überstehende plättchenreiche Plasma (PRP) abgenommen. Das Restblut wird zur Gewinnung von Plasma nocheinmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose (3.700 Bq) und 50 µl $^3$H-BIBU 52 (Endkonzentration: 5 nM.) bei Raumtemperatur 20 Minuten inkubiert. Zur Messung der unspezifischen Bindung wird anstelle der Testsubstanz 5 µl BIBU 52 (Endkonzentration: 30 µM) eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0,2N NaOH gelöst, 450 µl werden mit 2 ml Szintillator und 25 µl 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testssubstanzaufgetragen und die Konzentration für eine 50%ige Hemmung der Bindung ermittelt.

2. Antithrombotische Wirkung

Methodik

Die Thrombozytenaggregation wird nach der Methode von Born und Cross (J. Physiol. 170, 397 (1964)) in plättchenreichem
Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Konzentration wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37°C inkubiert.

Aus den Konzentrations-Wirkungskurven wird die $EC_{50}$ berechnet, die die Konzentration beschreibt, bei der die Änderung der "optical density" halbmaximal gehemmt ist.

Die nachfolgende Tabelle enthält die gefundenen Ergebnisse:

| Substanz (Beispiel-Nr.) | Kompetitive Bindung $^3$H-BIBU 52 Textsubstanz an Humanthrombozyten $IC_{50}$ [nM] | Hemmung der Plättchenaggregation $EC_{50}$[nM] |
|---|---|---|
| 2 | 24000,0 | 3000 |
| 2(3) | 550,0 | 380 |
| 3 | 400,0 | 880 |
| 3(1) | 6800,0 | 4400 |
| 3(3) | 1,6 | 40 |
| 5 | 510,0 | 320 |
| 5(3) | 11,0 | 100 |

Die erfindungsgemäßen Verbindungen sind gut verträglich, da beispielsweise bei intravenöser Gabe von 30 mg/kg an jeweils 3 Mäusen der Verbindungen der Beispiele 5 und 5(3) keine Tiere gestorben waren.

Außerdem erzielt man beispielsweise mit dem nach peroraler Gabe vom 10 mg/kg der Verbindung des Beispiels 2(19) gewonnenen Rattenplasma ex vivo nach 3 Stunden eine 80%ige Hemmung der durch Kollagen induzierten Aggregration von Humanthrombozyten.

Auf Grund ihrer Hemmwirkung auf Zell-Zell- bzw. Zell-Matrix-Wechselwirkungen eignen sich die neuen Carbonsäurederivate der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, vor zerebrovasculären Erkrankungen, von Lungenembolien, des Herzinfarktes, der Arteriosklerose, der Osteoporose, der Metastasierung von Tumoren und der Therapie genetisch bedingter oder auch erworbener Störungen der Interaktion von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 $\mu$g und 30 mg/kg Körpergewicht, vorzugsweise bei 1 $\mu$g bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, $\alpha$-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einen oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Sprays oder Zäpfchen einarbeiten.

Die nachfolgenden Beispiele sollen die Erfindung näher erläutern:

Beispiel I

4-(4-Piperidyl)-benzoesäure-hydrochlorid

Zu einer Lösung von 63,0 g 1-Acetyl-4-phenyl-piperidin in 1000 ml Methylenchlorid tropft man unter gutem Rühren bei -10 bis -20°C 157,4 g Oxalylchlorid. Anschließend gibt man 46,7 g Aluminiumchlorid zu. Man rührt 1 Stunde bei -10°C und gibt weitere 82,7 g Aluminiumchlorid zu. Nach weiteren 2 Stunden wird das Kühlbad entfernt und 24 Stunden bei Raumtemperatur gerührt. Die Reaktionslösung wird vorsichtig in

ca. 4 l Eis/Wasser eingerührt und die wäßrige Phase zweimal mit Methylenchlorid extrahiert. Die vereinigten organischen Phasen werden mit Wasser gewaschen, über Natriumsulfat getrocknet, und das Lösungsmittel unter vermindertem Druck entfernt. Der verbleibende Rückstand wird unter kräftigem Rühren in 2,5 l 2N Natronlauge gelöst. Zur dunklen wäßrigen Lösung gibt man Eis und säuert mit konz. Salzsäure an. Der Niederschlag wird abgesaugt, mit Wasser gewaschen und in 2 l 6N Salzsäure 5 Stunden zum Rückfluß erhitzt. Das Lösungsmittel wird unter vermindertem Druck entfernt. Der verbleibende Feststoff wird mit wenig Wasser verrieben und abgesaugt.
Ausbeute: 40,5 g (54 % der Theorie),
Schmelzpunkt: > 300 ° C
$R_f$-Wert: 0,07 (Kieselgel; Methylenchlorid/Methanol/konz. Ammoniak = 4:1:0,25)

Beispiel II

4-[1-(tert.Butyloxycarbonyl)-4-piperidyl]-benzoesäure

Zu 16,4 g Natriumhydroxid in 300 ml Wasser gibt man vorsichtig 47,5 g 4-(4-Piperidyl)-benzoesäure-hydrochlorid. Die Suspension wird mit 500 ml Dioxan und 250 ml Wasser verdünnt. Anschließend werden 54,6 g Pyrokohlensäure-di-tert.butylester portionsweise zugegeben. Man rührt 16 Stunden bei Raumtemperatur. Dar Niederschlag wird abgesaugt und das Filtrat unter vermindertem Druck teilweise eingedampft. Der Niederschlag und das verbleibende wäßrige Filtrat werden vereinigt und mit 1 l Wasser verdünnt. Die wäßrige Phase wird mit gesättigter Kaliumhydrogensulfat-Lösung auf pH 2 gebracht und zweimal mit Essigester extrahiert. Die vereinigten Essigester-Phasen werden mit jesättigter Kochsalzlösung gewaschen, über Natriumsulfat getrocknet und das Lösungsmittel unter vermindertem Druck entfernt. Das kristalline Rohprodukt wird mit wenig Essigester verrieben, abgesaugt und getrocknet.
Ausbeute: 54,0 g (90 % der Theorie),
Schmelzpunkt: 172-174 ° C
$R_f$-Wert: 0,73 (Kieselgel; Essigester/Cyclohexan = 4:1)

Beispiel III

4-[1-(tert.Butyloxycarbonyl)-4-piperidyl]-benzoesäure-hydrazid

Zu einer Lösung von 21,3 g 4-[1-(tert.Butyloxycarbonyl)-4-piperidyl]-benzoesäure in 150 ml Dimethylformamid gibt man bei -10 ° C 9,6 g 1-Hydroxy-(1H)-benzotriazol und 17,4 g N,N'-Dicyclohexylcarbodiimid. Man rührt 15 Minuten bei -10 ° C, entfernt das Kühlbad und läßt die Temperatur auf Raumtemperatur ansteigen. Diese Reaktionslösung tropft man anschließend zu einer auf -10 ° C gekühlten Lösung von 50 ml 80%igem Hydrazinhydrat in 150 ml Dimethylformamid. Man rührt 16 Stunden bei Raumtemperatur und saugt den Niederschlag ab. Das Filtrat wird unter vermindertem Druck eingeengt. Zum Rückstand gibt man Wasser und extrahiert die wäßrige Phase dreimal mit Essigester. Die vereinigten organischen Phasen werden über Natriumsulfat getrocknet und das Lösungsmittel unter vermindertem Druck eingedampft. Der verbleibende Feststoff wird mit Essigester/Cyclohexan (4:1) über Kieselgel chromatographiert. Man erhält 18,5 g eines kristallinen Feststoffs, welcher mit Essigester verrieben und anschließend abgesaugt wird.
Ausbeute: 12,7 g (57 % der Theorie),
Schmelzpunkt: 151-154 ° C
$R_f$-Wert: 0,26 (Kieselgel; Essigester/Cyclohexan = 4:1)

Beispiel IV

N-[4-[1-(tert.Butyloxycarbonyl)-4-piperidyl]-benzoyl]-N'-[(methoxycarbonyl)-carbonyl]-hydrazin

Zu einer Lösung von 3,2 g 4-[1-(tert.Butyloxycarbonyl)-4-piperidyl]-benzoesäure-hydrazid und 1,7 g Ethyldiisopropylamin in 50 ml wasserfreiem Tetrahydrofuran tropft man unter Kühlung im Eisbad eine Lösung von 1,3 g Oxalsäure-monomethylester-chlorid in 10 ml wasserfreiem Tetrahydrofuran. Man rührt 16 Stunden bei Raumtemperatur, saugt den Niederschlag ab und engt das Filtrat unter vermindertem Druck ein. Der Rückstand wird in Essigester gelöst und einmal mit 0,5 N Salzsäure gewaschen. Die organische Phase wird getrocknet und das Lösungsmittel unter vermindertem Druck abgedampft. Das Rohprodukt wird mit Essigester/Cyclohexan (4:1) über Kieselgel chromatographiert.
Ausbeute: 3,5 g (86 % der Theorie),

Schmelzpunkt: 105-108 °C
$R_f$-Wert: 0,27 (Kieselgel; Essigester/Cyclohexan = 4:1)
Analog werden folgende Verbindungen erhalten:

(1) N-[4-[1-(tert.Butyloxycarbonyl)-4-piperidyl]-benzoyl]-N'-[[4-(methoxycarbonyl)-butyl]-carbonyl]-hydrazin Es wird Adipinsäure-monomethylester-chlorid eingesetzt.
Schmelzpunkt: 150-153 °C
$R_f$-Wert: 0,26 (Kieselgel; Essigester/Cyclohexan = 4:1)

(2) N-[4-[4-(tert.Butyloxycarbonyl)-4-piperidyl]-benzoyl]-N'-[[cis-4-(methoxycarbonyl)-cyclohexyl]-carbonyl]-hydrazin Es wird cis-4-(Methoxycarbonyl)-cyclohexancarbonsäurechlorid eingesetzt.
$R_f$-Wert: 0,20 (Kieselgel; Methylenchlorid/Methanol = 20:1)

(3) N-[4-[4-(tert.Butyloxycarbonyl)-4-piperidyl]-benzoyl]-N'-[[trans-4-(methoxycarbonyl)-cyclohexyl]-carbonyl]-hydrazin Es wird ein cis/trans-Gemisch aus 4-(Methoxycarbonyl)-cyclohexancarbonsäure-chlorid eingesetzt. Das trans-Produkt fällt aus der organischen Phase aus.
Schmelzpunkt: 198-201 °C
$R_f$-Wert: 0,20 (Kieselgel; Methylenchlorid/Methanol = 20:1)

(4) N-[4-[1-(tert.Butyloxycarbonyl)-4-piperidyl]-benzoyl]-N'-[[trans-4-(ethoxycarbonyl)-cyclohexyl]-carbonyl]-hydrazin
$R_f$-Wert: 0,53 (Kieselgel; Methylenchlorid/Methanol = 9:1)

(5) N-[4-(1-Benzyl-4-piperazinyl)-benzoyl]-N'-[[trans-4-(ethoxycarbonyl)-cyclohexyl]-carbonyl[-hydrazin
Schmelzpunkt: 182-184 °C
$R_f$-Wert: 0,78 (Kieselgel; Methylenchlorid/Methanol = 9:1)

(6) N-[[1-[1-(tert.Butyloxycarbonyl)-4-piperidyl]-4-pipe-ridyl]carbonyl]-N'-[[trans-4-(ethoxycarbonyl)-cyclohexyl]-carbonyl]-hydrazin
Schmelzpunkt: 222-224 °C (Zers.)
$R_f$-Wert: 0,79 (Kieselgel; Methylenchlorid/Methanol = 9:1)

## Beispiel V

### 2-[4-[1-(tert.Butyloxycarbonyl)-4-piperidyl]-phenyl]-5-methoxycarbonyl-1,3,4-thiadiazol

Eine Lösung von 3,32 g N-[4-[1-(tert.Butyloxycarbonyl)-4-piperidyl]-benzoyl]-N-[(methoxycarbonyl)-carbonyl]-hydrazin und 3,64 g 2,4-Bis-(4-methoxyphenyl)-1,3-dithia-2,4-diphosphetan-2,4-disulfid (Lawesson's Reagenz) in 60 ml Tetrahydrofuran wird 30 Minuten zum Rückfluß erwärmt. Das Lösungsmittel wird unter vermindertem Druck entfernt und der Rückstand mit Essigester/Cyclohexan (2:1) über Kieselgel chromatographiert.
Ausbeute: 2,75 g (83 % der Theorie),
Schmelzpunkt: 143-146 °C
$R_f$-Wert: 0,59 (Kieselgel; Essigester/Cyclohexan = 1:1)

## Beispiel VI

### 2-[4-[1-(tert.Butyloxycarbonyl)-4-piperidyl]-phenyl]-5-carboxy-1,3,4-thiadiazol

Eine Lösung von 2,7 g 2-[4-[1-(tert.Butyloxycarbonyl)-4-piperidyl]-phenyl]-5-methoxycarbonyl-1,3,4-thiadiazol und 1,1 g Lithiumhydroxid-hydrat in 50 ml Tetrahydrofuran und 40 ml Wasser wird 30 Minuten bei Raumtemperatur gerührt. Die Reaktionslösung wird mit 1N Salzsäure neutralisiert (pH 6,3). Das Lösungsmittel wird unter vermindertem Druck eingedampft und der Rückstand mit wenig Wasser verrieben und abgesaugt.
Ausbeute: 2,25 g (87 % der Theorie),
$R_f$-Wert: 0,42 (Kieselgel; Methylenchlorid/Methanol/konz. Ammoniak = 4:1:0,25)

## Beispiel VII

### 4-[2-Amino-2-(methoxycarbonyl)-ethyl]-1-[6-(4-cyan-phenyl)-3-pyridazinyl]-imidazol-dihydrochlorid

Zu einer Suspension von 1,55 g einer 55%igen Dispersion von Natriumhydrid in Mineralöl in 50 ml wasserfreiem Dimethylformamid tropft man unter Stickstoffatmosphäre bei 0 °C eine Lösung von 9,6 g N-a-tert.Butyloxycarbonyl-L-histidin-methylester in 100 ml trockenem Dimethylformamid. Man rührt 30 Minuten

bei 0 °C und tropft anschließend eine Lösung von 7,6 g 3-Chlor-6-(4-cyan-phenyl)-pyridazin in 400 ml trockenem Dimethylformamid zu. Man rührt 2 Stunden bei 0 °C und 16 Stunden bei Raumtemperatur. Die Reaktionslösung wird auf eine Eis/Kochsalzlösung gegossen und die wäßrige Phase dreimal mit Essigester extrahiert. Die vereinigten organischen Phasen werden über Natriumsulfat getrocknet und das Lösungsmittel unter vermindertem Druck abgedampft. Der Rückstand wird mit Methylenchlorid/Methanol (20:1) über Kieselgel chromatographiert. Nach Entfernen des Lösungsmittels wird das so erhaltene Rohprodukt (11,0 g) in 500 ml Dioxan gelöst. Man gibt 150 ml mit Chlorwasserstoff gesättigten Ether zu und saugt den ausgefallenen Niederschlag ab. Der Feststoff wird in einer Mischung aus 750 ml Dioxan, 750 ml Methanol und 250 ml mit Chlorwasserstoff gesättigtem Ether gelöst und 16 Stunden bei Raumtemperatur gerührt. Anschließend wird der Niederschlag abgesaugt und mit Dioxan gewaschen (Ausbeute 4,4 g). Durch Einengen der Mutterlauge auf ca. 100 ml und Absaugen des Niederschlags werden weitere 4,7 g Produkt erhalten.
Gesamtausbeute: 9,1 g (61 % der Theorie),
Schmelzpunkt: Sinterung ab 220 °C
$R_f$-Wert: 0,43 (Kieselgel; Methylenchlorid/Methanol/konz. Ammoniak = 9:1:0,1)

Beispiel VIII

4-[2-(n-Butansulfonylamino)-2-(methoxycarbonyl)-ethyl]-1-[6-(4-cyan-phenyl)-3-pyridazinyl]-imidazol

Zu einer Lösung von 4,2 g 4-[2-Amino-2-(methoxycarbonyl)-ethyl]-1-[6-(4-cyan-phenyl)-3-pyridazinyl]-imidazol-dihydrochlorid und 4,5 g Ethyldiisopropylamin in 150 ml Methylenchlorid tropft man bei 0 °C eine Lösung von 2,0 g n-Butansulfonylchlorid in 20 ml Dimethylformamid. Man rührt 2 Tage bei Raumtemperatur und saugt anschließend den Niederschlag ab. Das Filtrat wird zweimal mit Wasser gewaschen. Die organische Phase wird über Natriumsulfat getrocknet und das Lösungsmittel unter vermindertem Druck abgedampft. Der Rückstand wird mit wenig Methanol verrieben und abgesaugt.
Ausbeute: 0,85 g (18 % der Theorie),
$R_f$-Wert: 0,49 (Kieselgel; Methylenchlorid/Methanol/konz. Ammoniak = 9:1:0,1)

Beispiel IX

1-(tert.Butyloxycarbonyl)-4-(2-cyano-ethyl)-piperidin

Eine Suspension von 4,61 g 1-(tert.Butyloxycarbonyl)-4-[2-(methansulfonyl)-ethyl]-piperidin, 10,5 g Kaliumcyanid und einer Spatelspitze Natriumiodid in 10 ml wasserfreiem Dimethylformamid wird 16 Stunden bei Raumtemperatur gerührt. Nach Zugabe von einigen ml Dimethylformamid wird 7 Stunden auf 70 °C erwärmt. Zur abgekühlten Suspension gibt man Eiswasser und 3 ml 2N Natronlauge und extrahiert die wäßrige Phase mehrmals mit tert.Butyl-methyl-ether. Die vereinigten organischen Phasen werden mit Eiswasser und gesättigter Kochsalzlösung gewaschen und über Natriumsulfat getrocknet. Das Lösungsmittel wird unter vermindertem Druck abgedampft.
Ausbeute: 3,1 g (86 % der Theorie), gelbliches Öl
$R_f$-Wert: 0,63 (Kieselgel; Cyclohexan/Eissigester = 1:1)

Beispiel X

1-(tert.Butyloxycarbonyl)-4-[2-(aminothiocarbonyl)-ethyl]-piperidin

Durch eine Lösung von 3,0 g 1-(tert.Butyloxycarbonyl)-4-(2-cyano-ethyl)-piperidin in 15 ml Pyridin und 1,15 ml Triethylamin leitet man bei einer Temperatur von -5 bis 0 °C für einige Minuten Schwefelwasserstoff. Die Reaktionslösung wird 16 Stunden bei 0 °C und weitere 24 Stunden bei Raumtemperatur gerührt. Anschließend wird während 3 Stunden Stickstoff durch die Reaktionslösung geleitet. Man gießt die Lösung in 150 ml Eis/Wasser-Gemisch und extrahiert die wäßrige Phase zweimal mit tert.Butyl-methyl-ether. Die vereinigten organischen Extrakte werden nacheinander zweimal mit Wasser, einmal mit 2N Citronensäurelösung, einmal mit Wasser und einmal mit gesättigter Kochsalzlösung gewaschen. Die organische Phase wird getrocknet und das Lösungsmittel unter vermindertem Druck abgedampft. Man erhält ein gelbes Öl, welches mit wenig Cyclohexan verrührt wird. Nach Zugabe von Petrolether wird im Eis/Wasser-Bad abgekühlt und das ausgefallene farblose Kristallisat abgesaugt.
Ausbeute: 550 mg (16 % der Theorie),

Schmelzpunkt: 148-154 °C,
$R_f$-Wert: 0,28 (Kieselgel; Cyclohexan/Eissigester = 1:1)
Analog Beispiel X wird folgende Verbindung erhalten:
(1) 4-(Benzyloxy)-benzoesäure-thioamid
Man gießt die Reaktionslösung in Wasser und saugt den Niederschlag ab. Der Feststoff wird in Essigester gelöst und die Lösung über Natriumsulfat getrocknet. Nach dem Einengen der Lösung unter vermindertem Druck fällt das Produkt aus.
Schmelzpunkt: 172-174 °C
$R_f$-Wert: 0,57 (Kieselgel; Methylenchlorid/Essigester = 9:1)

Beispiel XI

3-[4-(2-Chlor-acetyl)-phenyl]-propionsäure-ethylester

Zu 56,0 g Aluminiumtrichlorid in 150 ml Dichlorethan tropft man bei 0 °C 23,7 g Chloracetylchlorid. Anschließend tropft man 35,6 g 3-Phenyl-propionsäure-ethylester zu, wobei die Temperatur zwischen -5 und 5 °C gehalten wird. Man rührt 3 Stunden bei Raumtemperatur. Die Reaktions-Suspension gießt man in ein Gemisch aus Eis und 30 ml konzentrierte Salzsäure, trennt die Phasen und extrahiert die wäßrige Phase zweimal mit Chloroform. Die vereinigten organischen Phasen werden mit Wasser gewaschen und über Magnesiumsulfat getrocknet. Das Lösungsmittel wird unter vermindertem Druck abgedampft, mit Petrolether verrührt und abgesaugt.
Ausbeute: 44,2 g (87 % der Theorie),
$R_f$-Wert: 0,73 (Kieselgel; Cyclohexan/Eissigester = 1:1)

Beispiel XII

2-[4-(Benzyloxy)-phenyl]-4-[2-(methoxycarbonyl)-ethyl]-1,3-thiazol

Eine Lösung von 14,0 g 4-(Benzyloxy)-benzoesäure-thioamid und 12,0 g 4-Brom-3-oxo-butan-carbonsäure-methylester in 1000 ml Methanol wird 24 Stunden zum Rückfluß erhitzt. Das Lösungsmittel wird unter vermindertem Druck abgedampft und der Rückstand zwischen Methylenchlorid und verdünnter Natriumcarbonatlösung verteilt. Die organische Phase wird einmal mit verdünnter Natriumcarbonat-Lösung und einmal mit Wasser ausgeschüttelt, über Natriumsulfat getrocknet und das Lösungsmittel unter vermindertem Druck eingedampft. Der Rückstand wird aus Methanol umkristallisiert.
Ausbeute: 15,0 g (74 % der Theorie),
Schmelzpunkt: 80 °C
$R_f$-Wert: 0,25 (Kieselgel; Methylenchlorid/Essigester = 19:1)

Beispiel XIII

4-(2-Carboxy-ethyl)-2-(4-hydroxy-phenyl)-1,3-thiazol

Eine Lösung von 8,0 g 2-[4-(Benzyloxy)-phenyl]-4-[2-(methoxycarbonyl)-ethyl]-1,3-thiazol in 200 ml Eisessig und 40 ml mit Chlorwasserstoff gesättigtem Methanol wird in Gegenwart von 4,0 g 10 % Palladium auf Kohle 1,5 Stunden bei Raumtemperatur und einem Wasserstoffdruck von 5 bar hydriert. Der Katalysator wird abfiltriert und das Filtrat unter vermindertem Druck eingedampft. Der Rückstand wird mit Essigester zum Sieden erhitzt, abgekühlt und der Niederschlag abgesaugt.
Ausbeute: 5,4 g (96 % der Theorie),
Schmelzpunkt: 224-226 °C
$R_f$-Wert: 0,53 (Kieselgel; Toluol/Dioxan/Ethanol/Eisessig = 9:1:1:0,6)

Beispiel XIV

2-(4-Hydroxy-phenyl)-4-[2-(methoxycarbonyl)-ethyl]-1,3-thiazol

Eine Lösung von 5,0 g 4-(2-(Carboxy-ethyl)-2-(4-hydroxy-phenyl)-1,3-thiazol in 100 ml Methanol und 10 ml mit Chlorwasserstoff gesättigtem Methanol wird 16 Stunden bei Raumtemperatur gerührt. Das Lösungsmittel wird unter vermindertem Druck eingedampft. Der Rückstand wird in ca. 100 ml Wasser auf dem

Dampfbad erhitzt, anschließend im Eisbad abgekühlt und der Niederschlag abgesaugt.
Ausbeute: 4,2 g (79 % der Theorie),
Schmelzpunkt: 103-105 °C
$R_f$-Wert: 0,33 (Kieselgel; Cyclohexan/Essigester = 2:1)

Beispiel XV

4-[4-[2-(Ethoxycarbonyl)-ethyl]-phenyl]-2-methyl-imidazol

Eine Suspension von 5,1 g 3-[4-(2-Chlor-acetyl)-phenyl]-propionsäure-ethylester, 2,3 g Acetamidin-hydrochlorid und 4,7 g Natriumcarbonat in 20 ml wasserfreiem Ethanol wird 2 Stunden zum Rückfluß erhitzt. Das Lösungsmittel wird unter vermindertem Druck abgedampft und der Rückstand zwischen Essigester und Wasser verteilt. Die organische Phase wird dreimal mit Wasser gewaschen, über Magnesiumsulfat getrocknet und das Lösungsmittel unter vermindertem Druck abgedampft. Der Rückstand wird mit Cyclohexan/Essigester (3:7) über Aluminiumoxid der Aktivitätsstufe II chromatographiert.
Ausbeute: 900 mg (17 % der Theorie)
$R_f$-Wert: 0,23 (AloxN; Esssigester/Cyclohexan = 7:3)

Beispiel XVI

[4-(1-Benzyl-4-piperazinyl)-benzoyl]-hydrazin

3 g N-[4-(1-Benzyl-4-piperazinyl)-benzoyl]-N'-(tert.butyloxycarbonyl)-hydrazin werden in 30 ml Methylenchlorid gelöst, mit 15 ml Trifluoressigsäure versetzt und 4 Stunden bei Raumtemperatur gerührt. Man engt ein, dampft zweimal mit Aceton nach und verteilt den Rückstand zwischen 2N Natronlauge und Essigester. Die Essigesterphasen werden eingeengt und der Rückstand über Kieselgel gereinigt (Elutionsmittel: Methylenchlorid/Methanol = 40:1 bis 25:1).
Ausbeute: 0,82 g (36% der Theorie),
Schmelzpunkt: 168-170 °C
$R_f$-Wert: 0,51 (Kieselgel; Methylenchlorid/Methanol = 9:1)

Beispiel XVII

N-[4-(1-Benzyl-4-piperazinyl)-benzoyl]-N'-(tert.butyloxycarbonyl -hydrazin

3 g 4-(1-Benzyl-4-piperazinyl)-benzoesäure werden unter Erwärmen in 100 ml Dimethylformamid gelöst und mit 1,05 ml Chlordiphenylphosphin versetzt. Man kühlt ab, fügt 1,5 ml Triethylamin zu, rührt eine Stunde unter Eiskühlung, versetzt mit 1,4 g tert.Butyloxycarbonyl-hydrazin, rührt 5 Tage bei Raumtemperatur und engt ein. Der Rückstand wird mit Essigester versetzt und mit Wasser ausgeschüttelt, wobei man das gebildete Festprodukt abfiltriert. Die Essigesterphasen werden mit Natriumbicarbonatlösung gewaschen und eingeengt. Der Rückstand wird mit Essigester/Petrolether kristallin gerieben. Ausbeute: 0,94 g (23% der Theorie),
Schmelzpunkt: 162-165 °C
$R_f$-Wert: 0,45 (Kieselgel; Methylenchlorid/Methanol = 9:1)

Beispiel XVIII

4-(1-Benzyl-4-piperazinyl)-benzoesäure

Eine Mischung aus 29,8 g 4-(1-Benzyl-4-piperazinyl)-benzoesäurenitril, 48 g Kaliumhydroxid und 200 ml Glycol wird 8 Stunden zum Rückfluß erhitzt. Das Glycol wird im Vakuum weitgehend abdestilliert, der Rückstand mit Wasser versetzt mit Eisessig angesäuert und das ausgefallene Produkt abfiltriert und mit Aceton gewaschen.
Ausbeute: 31,4 g (99% der Theorie),
Schmelzpunkt: 225-227 °C
$R_f$-Wert: 0,57 (Kieselgel; Methylenchlorid/Methanol = 9:1)

Beispiel XIX

4-(1-Benzyl-4-piperazinyl)-benzoesäure-nitril

26 g 4-Fluor-benzonitril, 37,3 ml N-Benzylpiperazin und 36,7 ml N-Ethyl-diisopropylamin werden zusammen 8 Stunden auf 140 °C erwärmt. Man gießt auf Wasser und extrahiert mit Methylenchlorid. Die organische Phase wird eingedampft und der Rückstand mit Ether/Petrolether kristallin gerieben.
Ausbeute: 29,8 g (50% der Theorie),
Schmelzpunkt: 106-108 °C
$R_f$-Wert: 0,89 (Kieselgel; Methylenchlorid/Methanol = 9:1)

Beispiel XX

[[1-[1-(tert.Butyloxycarbonyl)-4-piperidyl]-4-piperidyl]-carbonyl]-hydrazin

1 g 1-[1-(tert.Butyloxycarbonyl)-4-piperidyl]-4-ethoxycarbonyl-piperidin und 10 ml Hydrazinhydrat werden in 20 ml Methanol 4 Stunden zum Rückfluß erhitzt. Man engt ein und verreibt den Rückstand mit Ether.
Ausbeute: 0,72 g (76% der Theorie),
$R_f$-Wert: 0,42 (Kieselgel; Methylenchlorid/Methanol = 4:1)

Beispiel XXI

1-[1-(tert.Butyloxycarbonyl)-4-piperidyl]-4-ethoxycarbonyl-piperidin

Eine Mischung aus 24,9 g 1-tert.Butyloxycarbonyl-4-piperidon, 19,3 g Piperidin-4-carbonsäure-ethylester und 46,5 ml Titan(IV)-isopropylat wird 1 Stunde bei Raumtemperatur gerührt, mit 170 ml Ethanol und 5 g Natriumcyanborhydrid versetzt und 20 Stunden bei Raumtemperatur gerührt. Man fügt 34 ml Wasser zu, filtriert ab, engt das Filtrat ein, extrahiert mit Essigester und dampft diesen ein. Der Rückstand wird chromatographisch gereinigt (Kieselgel, Elutionsmittel:
Cyclohexan/Essigester = 2:3).
Ausbeute: 32,9 g (77% der Theorie),
$R_f$-Wert: 0,44 (Kieselgel; Essigester)

Beispiel 1

2-[4-[1-(tert.Butyloxycarbonyl)-4-piperidyl]-phenyl]-5-[[2-(methoxycarbonyl)-ethyl]-aminocarbonyl]-1,3,4-thiadiazol

Eine Lösung von 2,0 g 2-[4-[1-(tert.Butyloxycarbonyl)-4-piperidyl]-phenyl]-5-carboxy-1,3,4-thiadiazol, 1,77 g 2-[(1H)-Benzotriazol-1-yl]-1,1,3,3-tetramethyluronium-tetrafluorborat, 0,72 g b-Alaninmethylester-hydrochlorid, 0,75 g 1-Hydroxy-(1H-)benzotriazol und 1,4 g N-Methyl-morpholin in 50 ml Dimethylformamid wird 5 Stunden bei Raumtemperatur gerührt. Das Lösungsmittel wird unter vermindertem Druck entfernt. Zum Rückstand gibt man Wasser und extrahiert die wäßrige Phase mit Essigester. Die organische Phase wird über Natriumsulfat getrocknet und das Lösungsmittel unter vermindertem Druck entfernt. Das Rohprodukt wird mit Methylenchlorid/Methanol (9:1) über Kieselgel chromatographiert und das nach Entfernen des Lösungsmittels erhaltene Produkt mit Essigester verrieben und abgesaugt.
Ausbeute: 1,5 g (62 % der Theorie),
Schmelzpunkt: 176-178 °C,
$R_f$-Wert: 0,76 (Kieselgel; Eissigester/Cyclohexan = 4:1)

Beispiel 2

2-[[2-(Methoxycarbonyl)-ethyl]-aminocarbonyl]-5-[4-(4-piperidyl)-phenyl]-1,3,4-thiadiazol-hydrochlorid

Eine Lösung von 1,4 g 2-[4-[1-(tert.Butyloxycarbonyl)-4-piperidyl)-phenyl]-5-[[2-(methoxycarbonyl)-ethyl]-aminocarbonyl]-1,3,4-thiadiazol in 40 ml Dioxan und 40 ml mit Chlorwasserstoff gesättigtem Ether wird 2,5 Stunden bei Raumtemperatur gerührt. Das ausgefallene Produkt wird abgesaugt und mit Ether gewaschen.

Ausbeute: 1,05 g (85 % der Theorie),
Schmelzpunkt: 250-255 °C,
Massenspektrum: $M^+$ = 374
$R_f$-Wert: 0,16 (Kieselgel; Methylenchlorid/Methanol/konz. Ammoniak = 9:1:0,1)
Analog werden folgende Verbindungen erhalten:

(1) 2-[4-(Methoxycarbonyl)-butyl]-5-[4-(4-piperidyl)-phenyl]-1,3,4-thiadiazol-hydrochlorid
Massenspektrum: $(M+H)^+$ = 360
$R_f$-Wert: 0,70 (Kieselgel; Methylenchlorid/Methanol/konz. Ammoniak = 4:1:0,25)

(2) 2-[cis-4-(Methoxycarbonyl)-cyclohexyl]-5-[4-(4-piperidyl)-phenyl]-1,3,4-thiadiazol-hydrochlorid
Der Reaktionslösung wird mit Chlorwasserstoff gesättigtes Methanol zugesetzt. Nach einer Stunde Rühren bei Raumtemperatur wird das Lösungsmittel unter vermindertem Druck abgedampft. Massenspektrum: $M^+$ = 385
$R_f$-Wert: 0,45 (Kieselgel; Methylenchlorid/Methanol/konz. Ammoniak = 4:1:0,25)

(3) 2-[trans-4-(Methoxycarbonyl)-cyclohexyl)-5-[4-(4-piperidyl)-phenyl]-1,3,4-thiadiazol-hydrochlorid
Die Umsetzung wird in einem 1:1:1-Gemisch aus Dioxan, mit Chlorwasserstoff gesättigtem Ether und mit Chlorwasserstoff gesättigtem Methanol durchgeführt. Nach drei Stunden Rühren wird das ausgefallene Produkt abgesaugt und mit Ether gewaschen.
Massenspektrum: $M^+$ = 385
$R_f$-Wert: 0,67 (Kieselgel; Methylenchlorid/Methanol/konz. Ammoniak = 4:1:0,25)

(4) 2-[trans-4-(Methoxycarbonyl)-cyclohexyl]-4-[4-(4-piperidyl)-phenyl]-imidazol-dihydrochlorid

(5) 2-[trans-4-(Methoxycarbonyl)-cyclohexyl]-4-[3,4-dehydro4-piperidyl]-phenyl]-1,3-thiazol-hydrochlorid

(6) 2-[[3-(Methoxycarbonyl)-propyl]-amino]-4-[4-(4-piperidyl)-phenyl]-1,3-thiazol-dihydrochlorid

(7) 2-[trans-4-(Methoxycarbonyl)-cyclohexyl]-5-[4-(1-piperazinyl)-phenyl]-1,3,4-thiadiazol-dihydrochlorid

(8) 4-[4-[2-(Ethoxycarbonyl)-ethyl]-phenyl]-2-[2-(4-piperidyl)-ethyl]-1,3-thiazol-hydrochlorid

(9) 4-[4-[2-(Methoxycarbonyl)-ethyl]-phenyl]-2-[2-(4-piperidyl)-ethyl]-imidazol-dihydrochlorid

(10) 4-[4-[2-(Methoxycarbonyl)-ethyl]-phenyl]-1-methyl-2-[2-(4-piperidyl)-ethyl]-imidazol-dihydrochlorid

(11) 4-[4-[2-(Ethoxycarbonyl)-ethyl]-phenyl]-2-methyl-1-[2-(4-piperidyl)-ethyl]-imidazol-dihydrochlorid-hydrat
Die Umsetzung wird in Ethanol unter Zusatz von etherischer Salzsäure durchgeführt.
$R_f$-Wert: 0,48 (Reversed Phase; RP8; Methanol/5%ige Kochsalzlösung = 6:4)

| | | | | | | | | |
|---|---|---|---|---|---|---|---|---|
| Ber. x 1,95 HCl x $H_2O$ | C | 57,82 | H | 7,70 | N | 9,15 | Cl | 14,95 |
| Gef. | | 57,62 | | 7,68 | | 9,16 | | 15,07 |

(12) 2-[4-[2-(Methoxycarbonyl)-ethyl]-phenyl]-1-methyl-4-[(3-pyrrolidinyl)-oxymethyl]-imidazol-dihydrochlorid

(13) 4-[4-[2-(Methoxycarbonyl)-ethyl]-2-[N-[(4-piperidyl)-carbonyl]-N-methyl-amino]-1,3-thiazol-hydrochlorid

(14) 2-[4-[2-(Methoxycarbonyl)-ethyl]-phenyl]-4-[(4-piperidyl)-aminocarbonyl]-1,3-thiazol-hydrochlorid

(15) 2-[2-(Methoxycarbonyl)-ethyl]-4-[4-[N-[(4-piperidyl)-carbonyl]-N-methyl-amino]-phenyl]-1,3-thiazol-hydrochlorid

(16) 2-[2-(Methoxycarbonyl)-ethyl]-5-[2-[2-(4-piperidyl)-ethyl]-5-pyrimidinyl]-1,3,4-thiadiazol-hydrochlorid

(17) 4-[2-(Methoxycarbonyl)-ethyl]-2-[4-[(4-piperidyl)-methyloxy]-phenyl]-1,3-thiazol-dihydrochlorid-dihydrat
Die Umsetzung wird in mit Chlorwasserstoff gesättigtem Methanol durchgeführt. Der Niederschlag wird abgesaugt und mit tert.Butyl-methyl-ether gewaschen.
Schmelzpunkt: 95-98 °C
$R_f$-Wert: 0,28 (Reversed Phase; RP8; Methanol/5%ige Kochsalzlösung = 6:4)

(18) 4-[4-[(Methoxycarbonyl)-methyloxy]-phenyl]-2-[2-(4-piperidyl)-ethyl]-1,3-thiazol-hydrochlorid

(19) 2-[trans-4-(Ethoxycarbonyl)-cyclohexyl]-5-[4-(4-piperidyl)-phenyl]-1,3,4-thiadiazol-hydrochlorid
Die Umsetzung wird in einem 2:1-Gemisch aus Methylenchlorid und Trifluoressigsäure durchgeführt. Das Produkt wird mit einem Gemisch aus Ethanol und etherischer Salzsäure behandelt. Schmelzpunkt: 274-280 °C (Zers.)
$R_f$-Wert: 0,41 (Kieselgel; Methylenchlorid/Methanol = 9:1)

(20) 2-[trans-4-(Ethoxycarbonyl)-cyclohexyl]-5-[1-(4-piperidyl)-4-piperidyl]-1,3,4-thiadiazol-hydrochlorid
Man verfährt analog (19).
Schmelzpunkt: > 320 °C

$R_f$-Wert: 0,67 (Reversed Phase Platte RP18; Methanol 5%ige Natriumchloridlösung = 6:4)

Beispiel 3

2-[(2-Carboxyethyl)-aminocarbonyl]-5-[4-(4-piperidyl)-phenyl]-1,3,4-thiadiazol-hydrochlorid

Eine Suspension von 550 mg 2-[[2-(Methoxycarbonyl)-ethyl]-aminocarbonyl]-5-[4-(4-piperidyl)-phenyl]-1,3,4-thiadiazol-hydrochlorid in 100 ml 6N Salzsäure wird 3 Stunden bei Raumtemperatur gerührt. Nach Zugabe von weiteren 500 ml 6N Salzsäure wird eine weitere Stunde gerührt. Das Lösungsmittel wird unter vermindertem Druck entfernt und das Rohprodukt mit wenig Wasser verrieben und abgesaugt.
Ausbeute: 480 mg (91 % der Theorie),
Massenspektrum: $M^+$ = 360
$R_f$-Wert: 0,06 (Kieselgel; Methylenchlorid/Methanol/konz. Ammoniak = 4:1:0,25)
Analog werden folgende Verbindungen erhalten:

(1) 2-(4-Carboxybutyl)-5-[4-(4-piperidyl)-phenyl]-1,3,4-oxadiazol
Das Rohprodukt wird chromatographisch gereinigt.
Massenspektrum: $M^+$ = 329
$R_f$-Wert: 0,35 (Kieselgel; Methylenchlorid/Methanol/konz. Ammoniak = 2:1:0,25)

(2) 2-(cis-4-Carboxycyclohexyl)-5-[4-(4-piperidyl)-phenyl]-1,3,4-thiadiazol-hydrochlorid
Schmelzpunkt: > 310 °C
Massenspektrum: $M^+$ = 371
$R_f$-Wert: 0,17 (Kieselgel; Methylenchlorid/Methanol/konz. Ammoniak = 4:1:0,25)

(3) 1-[6-(4-Amidino-phenyl)-3-pyridazinyl]-4-[2-(n-butan-sulfonylamino)-2-carboxy-ethyl]-imidazol-hydrochlorid
Massenspektrum: $(M+H)^+$ = 472
$R_f$-Wert: 0,20 (Kieselgel; Methylenchlorid/Methanol/konz. Ammoniak = 2:1:0,25)

(4) 4-[4-(2-Carboxy-ethyl)-phenyl]-2-[2-(4-piperidyl)-ethyl]-1,3-thiazol
Das Produkt des Beispiels 9 wird in 3N Salzsäure eine Stunde auf dem Dampfbad erwärmt.
Schmelzpunkt: Zersetzung ab 168 °C
$R_f$-Wert: 0,38 (Reversed Phase RP8; Methanol/5%ige Kochsalzlösung = 6:4)

(5) 2-(trans-4-Carboxycyclohexyl)-4-[4-(4-piperidyl)-phenyl]-imidazol-dihydrochlorid
(6) 2-(trans-4-Carboxycyclohexyl)-1-methyl-4-[4-(1-methyl-4-piperidyl)-phenyl]-imidazol-dihydrochlorid
(7) 2-(trans-4-Carboxycyclohexyl)-1-methyl-4-[4-(4-piperidyl)-phenyl]-imidazol-dihydrochlorid
(8) 2-(trans-4-Carboxycyclohexyl)-4-[4-(3,4-dehydro-4-piperidyl)-phenyl]-1,3-thiazol-hydrochlorid
(9) 2-[(3-Carboxypropyl)-amino]-4-[4-(4-piperidyl)-phenyl]-1,3-thiazol-dihydrochlorid
(10) 2-(trans-4-Carboxycyclohexyl)-5-[4-(1-piperazinyl)-phenyl]-1,3,4-thiadiazol-dihydrochlorid
(11) 4-[4-(2-Carboxy-ethyl)-phenyl]-2-[2-(4-piperidyl)-ethyl]-imidazol-dihydrochlorid
(12) 4-[4-(2-Carboxy-ethyl)-phenyl]-1-methyl-2-[2-(4-piperidyl)-ethyl]-imidazol-dihydrochlorid
(13) 4-[4-(2-Carboxy-ethyl)-phenyl]-2-methyl-1-[2-(4-piperidyl)-ethyl]-imidazol-dihydrochlorid-hydrat
$R_f$-Wert: 0,64 (Reversed Phase; RP8; Methanol/5%ige Kochsalzlösung = 6:4)

| | | | | | | | | |
|---|---|---|---|---|---|---|---|---|
| Ber. x 1,95 HCl x 0,6 $H_2O$: | C | 56,77 | H | 7,37 | N | 9,84 | Cl | 16,40 |
| Gef.: | | 56,77 | | 7,13 | | 9,93 | | 16,34 |

(14) 2-[4-(2-Carboxy-ethyl)-phenyl]-1-methyl-4-[(3-pyrrolidinyl)-oxymethyl]-imidazol-dihydrochlorid
(15) 4-[4]-(2-Carboxy-ethyl)-phenyl]-2-[N-[(4-piperidyl)-carbonyl]-N-methyl-amino]-1,3-thiazol-hydrochlorid
(16) 2-[4]-(2-Carboxy-ethyl)-phenyl]-4-[(4-piperidyl)-aminocarbonyl]-1,3-thiazol-hydrochlorid
(17) 2-(2-Carboxy-ethyl)-4-[4-[N-[(4-piperidyl)-carbonyl]-N-methyl-amino]-phenyl]-1,3-thiazol-hydrochlorid
(18) 2-(2-Carboxy-ethyl)-5-[2-[2-(4-piperidyl)-ethyl]-5-pyrimidinyl]-1,3,4-thiadiazol-hydrochlorid (19) 1-[3-(4-Amidino-phenyl)-6-pyridazinyl]-3-[2-carboxy-2-(phenylsulfonylamino)-ethyl]-indol-hydrochlorid
(20) 2-(4-Amidino-phenyl)-4-[4-[2-(acetylamino)-2-carboxyethyl]-phenyl]-5-methyl-1,3-thiazol-hydrochlorid
(21) 4-(4-Amidino-phenyl)-2-[4-[2-(methansulfonylamino)-2-carboxy-ethyl]-phenyl]-1-methyl-imidazol-hydrochlorid
(22) 4-(2-Carboxy-ethyl)-2-[4-[(4-piperidyl)-methyloxy]-phenyl]-1,3-thiazol-dihydrochlorid-hydrat
Schmelzpunkt: Zersetzung ab 225 °C
$R_f$-Wert: 0,36 (Reversed Phase, RP8; Methanol/5%ige Kochsalzlösung = 6:4)
(23) 4-[4-(Carboxymethyloxy)-phenyl]-2-[2-(4-piperidyl)-ethyl]-1,3-thiazol-hydrochlorid

(24) 4-[4-(2-Carboxy-ethyl)-phenyl]-2-[2-(4-pyridyl)-ethyl]-1,3-thiazol-hydrochlorid
(25) 4-[4-(2-Carboxy-ethyl)-cyclohexyl]-1-[2-(4-chinuclidinyl)-ethyl]-2-methyl-imidazol-dihydrochlorid
(26) 2-[4-(1-Benzyl-4-piperazinyl)-phenyl]-5-(trans-4-carboxycyclohexyl)-1,3,4-thiadiazol
Massenspektrum: $M^+$ = 462
$R_f$-Wert: 0,15 (Reversed Phase Platte RP18; Methanol/5%-ige Natriumchloridlösung = 6:4)
(27) 2-(trans-4-Carboxy-cyclohexyl)-5-[1-(4-piperidyl)-4-piperidyl]-1,3,4-thiadiazol
Schmelzpunkt: > 320 °C
$R_f$-Wert: 0,67 (Reversed Phase Platte RP18; Methanol 5%ige Natriumchloridlösung = 6:4)

Beispiel 4

2-[4-[1-(tert.Butyloxycarbonyl)-4-piperidyl)-phenyl]-5-[4-(methoxycarbonyl)-butyl]-1,3,4-thiadiazol

Eine Lösung von 2,4 g N-[4-[1-(tert.Butyloxycarbonyl)-4-piperidyl]-benzoyl]-N'-[[4-(methoxycarbonyl)-butyl]-carbonyl]-hydrazin und 2,1 g 2,4-Bis-(4-methoxyphenyl)-1,3-dithia-2,4-diphosphetan-2,4-disulfid (Lawesson's Reagenz) in 40 ml Tetrahydrofuran wird 30 Minuten zum Rückfluß erwärmt. Das Lösungsmittel wird anschließend unter vermindertem Druck abgedampft und der Rückstand über Kieselgel chromatographiert.
Ausbeute: 2,4 g (quantitativ),
Schmelzpunkt: 111-113 °C,
Massenspoktrum: $M^+$ = 459
$R_f$-Wert: 0,38 (Kieselgel; Essigester/Cyclohexan = 1:1)
Analog werden folgende Verbindungen erhalten:
(1) 2-[4-[1-(tert.Butyloxycarbonyl)-4-piperidyl)-phenyl]-5-[cis-4-(methoxycarbonyl)-cyclohexyl]-1,3,4-thiadiazol
Schmelzpunkt: 130-132 °C
Massenspektrum: $M^+$ = 485
$R_f$-Wert: 0,49 (Kieselgel; Essigester/Cyclohexan = 1:1)
(2) 2-[4-[1-(tert.Butyloxycarbonyl)-4-piperidyl]-phenyl]-5-[trans-4-(methoxycarbonyl)-cyclohexyl]-1,3,4-thiadiazol
Schmelzpunkt: 180-182 °C
$R_f$-Wert: 0,57 (Kieselgel; Cyclohexan/Essigester = 1:1)
(3) 2-[4-[4-(tert.Butyloxycarbonyl)-1-piperazinyl]-phenyl]-5-[trans-4-(methoxycarbonyl)-cyclohexyl]-1,3,4-thiadiazol
(4) 2-[4-[1-(tert.Butyloxycarbonyl)-4-piperidyl]-phenyl]-5-[trans-4-(ethoxycarbonyl)-cyclohexyl]-1,3,4-thiadiazol
Schmelzpunkt: 164-166 °C
$R_f$-Wert: 0,78 (Kieselgel; Methylenchlorid/Methanol = 9:1)
(5) 2-[trans-4-(Ethoxycarbonyl)-cyclohexyl]-5-[4-(1-benzyl-4-piperazinyl)-phenyl]-1,3,4-thiadiazol
Schmelzpunkt: 166-170 °C
$R_f$-Wert: 0,63 (Kieselgel; Methylenchlorid/Methanol = 9:1)
(6) 2-[1-[1-(tert.Butyloxycarbonyl)-4-piperidyl]-4-piperi-dyl]-5-[trans-4-(ethoxycarbonyl)-cyclohexyl]-1,3,4-thiadiazol
$R_f$-Wert: 0,49 (Kieselgel; Methylenchlorid/Methanol = 9:1)

Beispiel 5

2-(4-Carboxy-butyl)-5-[4-(4-piperidyl)-phenyl]-1,3,4-thiadiazol-hydrochlorid

Eine Lösung von 1,0 g 2-[4-(Methoxycarbonyl)-butyl]-5-[4-(4-piperidyl)-phenyl]-1,3,4-thiadiazol-hydrochlorid und 0,22 g Lithiumhydroxid-hydrat in 30 ml Tetrahydrofuran und 24 ml Wasser wird 60 Minuten bei Raumtemperatur gerührt. Die Reaktionslösung wird mit 1N Salzsäure angesäuert. Überschüssiges Tetrahydrofuran wird unter vermindertem Druck abgedampft und der Niederschlag abgesaugt und mit wenig Wasser gewaschen.
Ausbeute: 0,80 g (83 % der Theorie),
Massenspektrum: $M^+$ = 345
$R_f$-Wert: 0,18 (Kieselgel; Methylenchlorid/Methanol/konz. Ammoniak = 4:1:0,25)
Analog werden folgende Verbindungen erhalten:

(1) 3-(4-Carboxy-cyclohexyl]-4-phenyl-5-[4-(4-piperidyl)-phenyl]-1,2,4-triazol
Nach dem Ansäuern wird das Lösungsmittelgemisch unter vermindertem Druck abgedampft und das Rohprodukt über Kieselgel chromatographiert.
Massenspaktrum: $M^+$ = 430
$R_f$-Wert: 0,12 (Kieselgel; Methylenchlorid/Methanol/konz. Ammoniak = 4:1:0,25)
(2) 3-(4-Carboxy-butyl)-4-phenyl-5-[4-(4-piperidyl)-phenyl]-1,2,4-triazol
Nach dem Ansäuern wird das Lösungsmittelgemisch unter vermindertem Druck abgedampft und das Rohprodukt über Kieselgel chromatographiert.
Massenspektrum: $M^+$ = 404
$R_f$-Wert: 0,24 (Kieselgel; Methylenchlorid/Methanol/konz. Ammoniak = 2:1:0,25)
(3) 2-(trans-4-Carboxy-cyclohexyl)-5-[4-(4-piperidyl)-phenyl]-1,3,4-thiadiazol-hydrochlorid
Massenspektrum: $M^+$ = 371
$R_f$-Wert: 0,09 (Kieselgel; Methylenchlorid/Methanol/konz. Ammoniak = 4:1:0,25)
(4) 2-(4-Carboxy-cyclohexyl)-5-[4-(4-piperidyl)-phenyl]-1,3,4-oxadiazol
Nach dem Ansäuern wird das Lösungsmittelgemisch unter vermindertem Druck abgedampft und das Rohprodukt über Kieselgel chromatographiert.
Massenspektrum: $M^+$ = 355
$R_f$-Wert: 0,09 (Kieselgel; Methylenchlorid/Methanol/konz. Ammoniak = 4:1:0,25)
(5) 2-(trans-4-Carboxy-cyclohexyl)-5-[4-(2,2,6,6-tetramethyl-4-piperidyl)-phenyl]-1,3,4-thiazol-hydrochlorid
(6) 2-(trans-4-Carboxy-cyclohexyl)-4-[4-(4-cyano-4-piperidyl)-phenyl]-1-methyl-imidazol-dihydrochlorid

Beispiel 6

2-[4-(Methoxycarbonyl)-butyl]-5-[4-(4-piperidyl)-phenyl]-1,3,4-oxadiazol

Eine Lösung von 2,0 g N-[4-[1-(tert.Butyloxycarbonyl)-4-piperidyl]-benzoyl]-N'-[[4-(methoxycarbonyl)-butyl]-carbonyl]-hydrazin und 0,3 ml Pyridin in 18 ml Thionylchlorid wird 3 Stunden bei Raumtemperatur gerührt. Anschließend wird 45 Minuten zum Rückfluß erwärmt. Überschüssiges Thionylchlorid wird unter vermindertem Druck abgedampft, der Rückstand mit Toluol versetzt und das Lösungsmittel wieder unter verminderten Druck abgedampft. Der Rückstand wird über Kieselgel chromatographiert.
Ausbeute: 0,24 g (15 % der Theorie),
Massenspektrum: $M^+$ = 343
$R_f$-Wert: 0,16 (Kieselgel; Methylenchlorid/Methanol/konz. Ammoniak = 8:1:0,1)
Analog wird folgende Verbindung erhalten:
(1) 2-[4-(Methoxycarbonyl)-cyclohexyl]-5-[4-(4-piperidyl)-phenyl]-1,3,4-oxadiazol
Massenspektrum: $M^+$ = 369
$R_f$-Wert: 0,24 (Kieselgel; Methylenchlorid/Methanol/konz. Ammoniak = 9:1)

Beispiel 7

3-[4-(Methoxycarbonyl)-cyclohexyl]-4-phenyl-5-[4-(4-piperidyl)-phenyl]-1,2,4-triazol

Zu einer Lösung von 785 mg Anilin in 5 ml 1,2-Dichlorbenzol gibt man 220 mg Phosphortrichlorid und erwärmt kurzzeitig auf 60 °C. Anschließend gibt man 700 mg N-[4-[4-(tert.-Butyloxycarbonyl)-4-piperidy]-benzoyl]-N'-[[cis-4-(methoxycarbonyl)-cyclohexyl]-carbonyl]-hydrazin zu und erwärmt 2,5 Stunden zum Rückfluß. Das Lösungsmittel wird unter vermindertem Druck abgedampft. Zum Rückstand gibt man Wasser und soviel 1N Natronlauge, bis die Lösung alkalisch reagiert. Die wässrige Phase wird einmal mit Methylenchlorid und einmal mit Essigester extrahiert. Die vereinigten organischen Phasen werden über Natriumsulfat getrocknet und die Lösungsmittel unter vermindertem Druck abgedampft. Der Rückstand wird über Kieselgel chromatographiert.
Ausbeute: 550 mg (86 % der Theorie),
Massenspektrum: $(M+H)^+$ = 445
$R_f$-Wert: 0,36 (Kieselgel; Methylenchlorid/Methanol/konz. Ammoniak = 4:1:0,25)
Analog wird folgende Verbindung erhalten:
(1) 3-[4-(Methoxycarbonyl)-butyl]-4-phenyl-5-[4-(4-piperidyl)-phenyl]-1,2,4-triazol
$R_f$-Wert: 0,34 (Kieselgel; Methylenchlorid/Methanol/konz. Ammoniak = 4:1:0,25)

Beispiel 8

1-[6-(4-Amidino-phenyl)-3-pyridazinyl]-4-[2-(n-butansulfonylamino)-2-(methoxycarbonyl)-ethyl]-imidazol

In eine Lösung von 700 mg 4-[2-(n-Butansulfonylamino)-2-(methoxycarbonyl)-ethyl]-1-[6-(4-cyan-phenyl)-3-pyridazinyl]-imidazol in 250 ml wasserfreiem Methanol leitet man bei 0°C unter Rühren eine Stunde Chlorwasserstoff ein. Nach 16-stündigem Rühren bei Raumtemperatur wird das Lösungsmittel unter vermindertem Druck bei einer Badtemperatur von 30°C abgedampft. Der Rückstand wird in 50 ml wasserfreiem Methanol gelöst und nach Zugabe von 3 g Ammoniumcarbonat 4 Stunden bei Raumtemperatur gerührt. Der Niederschlag wird abgesaugt und mit wasserfreiem Methanol nachgewaschen. Das Filtrat wird unter vermindertem Druck eingedampft und der Rückstand über Kieselgel chromatographiert.
Ausbeute: 250 mg (34 % der Theorie),
Schmelzpunkt: 227-229°C
Massenspektrum: $(M+H)^+$ = 486
$R_f$-Wert: 0,09 (Kieselgel; Methylenchlorid/Methanol/konz. Ammoniak = 4:1:0,25)
Analog werden folgende Verbindungen erhalten:

(1) 1-[3-[4-Amidino-phenyl]-6-pyridazinyl]-3-[2-(methoxycarbonyl)-2-(phenylsulfonylamino)-ethyl]-indol
(2) 4-[4-[2-(Acetylamino)-2-(methoxycarbonyl)-ethyl]-phenyl]-2-(4-amidino-phenyl)-5-methyl-1,3-thiazol
(3) 4-[4-Amidino-phenyl)-1-methyl-2-[4-[2-(methansulfonylamino)-2-(methoxycarbonyl)-ethyl]-phenyl]-imidazol

Beispiel 9

2-[2-[1-(tert.Butyloxycarbonyl)-4-piperidyl]-ethyl]-4-[4-[2-(ethoxycarbonyl)-ethyl]-phenyl]-1,3-thiazol

Eine Lösung von 550 mg 1-(tert.Butyloxycarbonyl)-4-(2-thioamido-ethyl)-piperidin und 530 mg 3-[4-(2-Chlor-acetyl)-phenyl]-propionsäure-ethylester in 30 ml Methanol wird 14 Stunden zum Rückfluß erhitzt. Bei Raumtemperatur tropft man anschließend 500 mg Ethyldiisopropylamin und eine Lösung von 500 mg Pyrokohlensäure-di-tert.butylester in Ether zu. Man rührt 10 Minuten bei Raumtemperatur und entfernt das Lösungsmittel unter vermindertem Druck. Der Rückstand wird über Kieselgel chromatographiert.
Ausbeute: 300 mg (32 % der Theorie),
$R_f$-Wert: 0,26 (Kieselgel; Cyclohexan/Essigester = 8:2)

Beispiel 10

2-[4-[[1-(tert.Butyloxycarbonyl)-4-piperidyl]-methyloxy]-phenyl]-4-[2-(methoxycarbonyl)-ethyl]-1,3-thiazol

Zu einer Lösung von 4,09 g 2-(4-Hydroxy-phenyl)-4-[2-(methoxycarbonyl-ethyl]-1,3-thiazol in 1000 ml wasserfreiem Dimethylformamid gibt man 1,7 g Kalium-tert.butylat und rührt 15 Minuten bei Raumtemperatur. Man gibt 4,5 g 1-(tert.Butyloxycarbonyl)-4-(mesyloxymethyl)-piperidin zu und erwärmt 24 Stunden auf 60°C. Nach der Zugabe von weiteren 0,9 g Kaliumtert.butylat und 2,2 g 1-(tert.Butyloxy-carbonyl)-4-(mesyloxymethyl)-piperidin wird 2 Tage bei 60°C weitergerührt. Das Lösungsmittel wird unter vermindertem Druck abgedampft und der verbleibende Rückstand über Kieselgel chromatographiert.
Ausbeute: 4,3 g (68 % der Theorie),
Schmelzpunkt: 75-77°C
$R_f$-Wert: 0,70 (Kieselgel; Methylenchlorid/Essigester = 4:1)

Beispiel 11

1-[2-[1-(tert.Butyloxycarbonyl)-4-piperidyl]-ethyl]-4-[4-[2-(ethoxycarbonyl)-ethyl]-phenyl]-2-methyl-imidazol

Eine Suspension von 820 mg 4-[4-[2-(Ethoxycarbonyl)-ethyl]-phenyl]-2-methyl-imidazol und 140 mg 55%iges Natriumhydrid in Mineralöl in 5 ml Dimethylformamid wird 30 Minuten bei Raumtemperatur gerührt. Man gibt 980 mg 1-(tert.Butyloxycarbonyl)-4-[2-(methansulfonyloxy)-ethyl]-piperidin zu und rührt 2 Stunden bei Raumtemperatur. Das Lösungsmittel wird unter vermindertem Druck abgedampft und der Rückstand zwischen Wasser und Essigester verteilt. Nach dem Neutralisieren der wäßrigen Phase mit 2N Zitronensäure wird die wässrige Phase mehrmals mit Essigester extrahiert. Die vereinigten Essigesterphasen werden mit Wasser gewaschen, über Magnesiumsulfat getrocknet und das Lösungsmittel unter

vermindertem Druck abgedampft. Der Rückstand wird mit Cyclohexan/Essigester (1:1) über Aluminiumoxid der Aktivitätsstufe III chromatographiert.
Ausbeute: 650 mg (43 % der Theorie),
$R_f$-Wert: 0,29 (Aluminiumoxid N; Cyclohexan/Essigester = 1:1)

Beispiel 12

2-[trans-4-(Isobutyloxycarbonyl)-cyclohexyl]-5-[4-(4-piperidyl)phenyl]-1,3,4-thiadiazol-hydrochlorid

In eine gerührte Suspension von 0,5 g 2-(trans-4-Carboxycyclohexyl)-5-[4-(4-piperidyl)-phenyl]-1,3,4-thiadiazolhydrochlorid in 25 ml Isobutanol wird 1,5 Stunden Salzsäuregas geleitet. Man läßt 16 Stunden bei Raumtemperatur weiterrühren, engt ein, verreibt den Rückstand mit Aceton und saugt ab.
Ausbeute: 0,54 g (95% der Theorie),
Schmelzpunkt: 265-267 °C
$R_f$-Wert: 0,63 (Reversed Phase Platte RP18; Methanol/5%-ige Natriumchloridlösung = 6:4)
Analog wird folgende Verbindung erhalten:
(1) 2-[trans-4-(Isopropyloxycarbonyl)-cyclohexyl]-5-[4-(4-piperidyl)-phenyl]-1,3,4-thiadiazol-hydrochlorid
$R_f$-Wert: 0,67 (Kieselgel; Methylenchlorid/Methanol/konz. Ammoniak = 4:1:0,25)

Beispiel 13

2-(trans-4-Carboxy-cyclohexyl)-5-[4-(1-piperazinyl)-phenyl]-1,3,4-thiadiazol

0,08 g 2-[4-(1-Benzyl-4-piperazinyl)-phenyl]-5-(trans-4-carboxycyclohexyl)-1,3,4-thiadiazol werden in 30 ml Dimethylformamid in Gegenwart von 0,5 g Palladiumhydroxid auf Kohle 7 Tage bei Raumtemperatur mit Wasserstoff von 4 bar behandelt.
$R_f$-Wert: 0,29 (Reversed Phase Platte RP18; Methanol/5%-ige Natriumchloridlösung = 6:4)
Analog wird folgende Verbindung erhalten:
(1) 2-[trans-4-(Ethoxycarbonyl)-cyclohexyl]-5-[4(1-piperazinyl)-phenyl]-1,3,4-thiadiazol-hydrochlorid
$R_f$-Wert: 0,35 (Kieselgel; Methylenchlorid/Methanol = 9:1)
Massenspektrum: $M^+$ = 400

Beispiel 14

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke | ad 1,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 15

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 16

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 17

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 18

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung als (2) und (4) unter intensiver Mischung zugegeben.

Diese Pulvermischung wird auf einer Kapselabfüllmaschine in Hartgelatine-Steckkapseln Größe 3 abgefüllt.

Beispiel 19

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** 5-gliedrige Heterocyclen der allgemeinen Formel

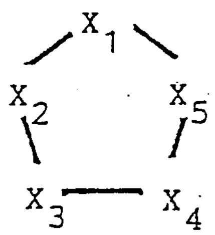

, (I)

in der

(i) mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält und

(ii) mit Ausnahme der Verbindungen 3-(4-Amidino-phenyl)-1-[4-(2-amino-2-carboxy-ethyl)-phenyl]-2H-pyrazol-5-on und 3-(4-Amidino-phenyl)-1-[4-(2-amino-2-methoxycarbonyl-ethyl)-phenyl]-2H-pyrazol-5-on,

einer der Reste $X_1$ bis $X_5$ eine Gruppe der Formeln

A - B - C - N< ,

A - B - C - CH< oder

A - B - C - C$\leqslant$ , in denen

A eine gegebenenfalls durch 1 bis 4 Alkylgruppen, durch eine Hydroxy-, Alkoxy-, Phenylalkoxy-, Cyano-, Aminocarbonyl-, Carboxy-, Alkoxycarbonyl- oder Phenylalkoxycarbonylgruppe substituierte Cycloalkylgruppe mit 5 bis 7 Kohlenstoffatomen, in der eine unsubstituierte Methylengruppe durch die $R_a$-N< Gruppe ersetzt ist, wobei

$R_a$ ein Wasserstoffatom, eine Alkylgruppe, eine Phenylalkylgruppe, eine Alkoxycarbonylgruppe mit insgesamt 2 bis 6 Kohlenstoffatomen, eine Phenylalkoxycarbonylgruppe, eine Alkenyloxycarbonylgruppe mit insgesamt 4 bis 6 Kohlenstoffatomen, eine Cycloalkoxycarbonylgruppe mit insgesamt 6 bis 8 Kohlenstoffatomen oder eine $R_1$-CO-O-($R_2$CH)-O-CO-Gruppe, in der

$R_1$ eine Alkylgruppe mit 1 bis 5 Kohlenstoffatomen, eine Cycloalkylgruppe mit 5 bis 7 Kohlenstoffatomen, eine Phenylalkylgruppe, eine Alkoxygruppe mit 1 bis 5 Kohlenstoffatomen, eine Cycloalkoxygruppe mit 5 bis 7 Kohlenstoffatomen oder eine Phenylgruppe und

$R_2$ ein Wasserstoffatom, eine Alkylgruppe mit 1 bis 4 Kohlenstoffatomen, eine Cycloalkylgruppe mit 5 bis 7 Kohlenstoffatomen oder eine Phenylgruppe darstellen,

und zusätzlich in den so gebildeten 6- oder 7-gliedrigen Azacycloalkylgruppen eine >CH- Einheit in 4-Stellung durch ein Stickstoffatom oder in den so gebildeten 5- bis 7-gliedrigen Azacycloalkylgruppen eine -$CH_2$-CH< Einheit durch eine -CH=C< Einheit und in den so gebildeten Piperazinyl- oder Homopiperazinylringen eine Methylengruppe, die benachbart zu dem Stickstoffatom in 4-Stellung steht, durch ein Carbonylgruppe ersetzt sein kann,

eine Pyridyl- oder Chinuclidinylgruppe oder auch, wenn

D eine durch eine $R_3R_4$N-CO-$NR_5$- oder $R_6$-$SO_2$-$NR_3$-Gruppe substituierte Alkylen- oder Alkenylengruppe mit jeweils bis zu 8 Kohlenstoffatomen, in denen

$R_3$ bis $R_5$, die gleich oder verschieden sein können, jeweils ein Wasserstoffatom, eine Alkyl- oder Phenylalkylgruppe und

$R_6$ eine Alkylgruppe mit 1 bis 5 Kohlenstoffatomen, eine Phenylalkyl- oder Phenylgruppe darstellen,

oder E eine durch eine Hydroxy-, Alkoxy-, Alkylsulfenyl-, $R_3R_4$N-, $R_3$O-CO-, $R_6$CO-$NR_3$-, $R_6$O-CO-$NR_3$-, $R_6SO_2$-$NR_3$- oder $R_3R_4$N-CO-$NR_5$-Gruppe substituierte geradkettige oder verzweigte Alkylen- oder Alkenylengruppe mit jeweils bis zu 5 Kohlenstoffatomen, in denen $R_3$ bis $R_6$ wie vorstehend erwähnt definiert sind, darstellen,

eine Phenylgruppe, die in 4-Stellung durch eine $R_a$NH-$CH_2$- oder $R_a$NH-C(=NH)-Gruppe substituiert ist, oder eine Phenylgruppe, an die über zwei benachbarte Kohlenstoffatome eine durch eine $R_a$NH-Gruppe substituierte n-Alkylenbrücke mit 3 oder 4 Kohlenstoffatomen angefügt ist, wobei $R_a$ jeweils wie vorstehend erwähnt definiert ist,

B eine Bindung, eine Alkylengruppe, eine -$OCH_2$-, -$CH_2$O-, -$SCH_2$-, -$CH_2$S-, -$CONR_3$-, -$R_3$NCO-, -$CH_2NR_3$-, -$NR_3CH_2$-, -$SO_2NR_3$- oder -$NR_3SO_2$-Gruppe, wobei $R_3$ wie vorstehend erwähnt definiert ist und ein Sauerstoff- oder Stickstoffatom des Restes B nicht direkt mit einem Stickstoffatom des Restes A oder ddes 5-gliedrigen Heterocyclus verbunden ist,

C eine Phenylengruppe, die durch Fluor-, Chlor- oder Bromatome, durch Alkyl-, Trifluormethyl-, Hydroxy-, Alkoxy-, Alkylsulfenyl-, Alkylsulfinyl- oder Alkylsulfonylgruppen mono- oder disubstituiert sein

kann, wobei die Substituenten gleich oder verschieden sein können,
eine Pyridinylen-, Pyrimidinylen-, Pyrazinylen- oder Pyridazinylengruppe, die jeweils im Kohlenstoffgerüst durch ein Chloratom, durch eine Alkyl- oder Alkoxygruppe substituiert sein können,
eine 1,4-Cyclohexylen-, 1,3-Piperidinylen-, 1,4-Piperidinylen- oder 1,4-Piperazinylengruppe oder auch, sofern nicht A eine Pyridylgruppe und gleichzeitig B eine Alkylen-, -CONH- oder $-CH_2S$-Gruppe darstellt, eine Bindung, wobei gleichzeitig B nur dann eine Bindung sein kann, wenn A eine Amidinophenylgruppe darstellt,
ein zweiter der Reste $X_1$ bis $X_5$ eine Gruppe der Formeln

$R_bO$-CO - E - D - N< ,
$R_bO$-CO - E - D - CK< oder
$R_bO$-CO - E - D - C$\leqslant$ , in denen

D eine $-CO-NR_3-$, $-NR_3-CO-$, $-SO_2-NR_3-$ oder $-NR_3-SO_2-$Gruppe, eine gegebenenfalls durch eine Hydroxy-, Alkoxy-, Alkylsulfenyl-, $R_3R_4N-$, $R_3O-CO-$, $R_6CO-NR_3-$, $R_6O-CO-NR_3-$, $R_6SO_2-NR_3-$ oder $R_3R_4N-CO-NR_5$-Gruppe substituierte geradkettige oder verzweigte Alkylen- oder Alkenylengruppe, in denen jeweils der Alkylenteil 1 bis 8 Kohlenstoffatome und der Alkenylenteil 2 bis 8 Kohlenstoffatome enthalten kann sowie $R_3$ bis $R_6$ wie vorstehend erwähnt definiert sind,
eine Phenylengruppe, die durch Fluor-, Chlor- oder Bromatome, durch Alkyl-, Trifluormethyl-, Hydroxy-, Alkoxy-, Alkylsulfenyl-, Alkylsulfinyl- oder Alkylsulfonylgruppen mono- oder disubstituiert sein kann,
eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine $-CO-NR_3$-Gruppe, in der $R_3$ wie vorstehend erwähnt definiert ist, ersetzt sein können und eines der Stickstoffatome statt an den Rest $R_3$ auch an den Rest E, sofern dieser keine Bindung darstellt und nicht über ein Sauerstoff- oder Schwefelatom an den Rest D gebunden ist, oder an das im Ring befindliche Atom des jeweiligen Restes $X_1$ bis $X_5$ gebunden sein kann,
eine gegebenenfalls durch eine Alkyl-, Phenylalkyl- oder Phenylgruppe substituierte Cycloalkylengruppe mit 4 bis 5 Kohlenstoffatomen, in der eine >CH-Einheit durch ein Stickstoffatom und zusätzlich eine zum Stickstoffatom benachbarte Methylengruppe durch eine Carbonylgruppe ersetzt sein kann,
eine gegebenenfalls durch eine Alkyl-, Phenylalkyl- oder Phenylgruppe substituierte Cycloalkylengruppe mit 6 oder 7 Kohlenstoffatomen, in der eine oder zwei >CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, oder
eine über den Rest $W_1$ mit dem im Ring befindlichen Atom des jeweiligen Restes $X_1$ bis $X_5$ verknüpfte Alkylengruppe mit 1 bis 5 Kohlenstoffatomen, in der $W_1$ eine $NR_3$-Gruppe, in der $R_3$ wie vorstehend erwähnt definiert ist, ein Sauerstoff- oder Schwefelatom darstellt, wobei ein Sauerstoff- oder Schwefelatom des Restes $W_1$ nicht direkt an ein Stickstoffatom des 5-gliedrigen Heterocyclus gebunden sein kann,
E eine Bindung, wobei nicht gleichzeitig C eine Bindung und A eine 1-Methyl- oder 1-Benzyl-4-piperazinylgruppe darstellen kann, oder
eine gegebenenfalls durch eine Hydroxy-, Alkoxy-, Alkylsulfenyl-, $R_3R_4N-$, $R_3O-CO-$, $R_6CO-NR_3-$, $R_6O-CO-NR_3-$, $R_6SO_2-NR_3-$ oder $R_3R_4N-CO-NR_5$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann sowie $R_3$ bis $R_6$ wie vorstehend erwähnt definiert sind, oder
eine über den Rest $W_2$ mit dem Rest D verknüpfte Alkylengruppe, in der $W_2$ ein Sauerstoff- oder Schwefelatom, eine Sulfinyl-, Sulfonyl-, $-NR_3-$, $-(R_6CO)N-$, $-(R_6SO_2)N-$, $-CONR_3-$ oder $-NR_3CO$-Gruppe darstellt, in denen $R_3$ und $R_6$ wie vorstehend definiert sind und wobei ein Sauerstoff- oder Schwefelatom des Restes $W_2$ nicht direkt an ein Stickstoffatom des Restes D gebunden ist, und
$R_b$ ein Wasserstoffatom, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil oder eine $R_1-CO-O-(R_2CH)$-Gruppe, in der $R_1$ und $R_2$ wie vorstehend erwähnt definiert sind, wobei der Abstand zwischen dem am weitesten entfernten Sticktoffatom der Gruppe A und der $COOR_b$-Gruppe mindestens 11 Bindungen beträgt,
ein dritter der Reste $X_1$ bis $X_5$ ein Schwefelatom, eine HN<, $R_6N$<, $R_7C\leqslant$ oder $(R_7)_2C$< Gruppe oder ein N-Atom, wobei $R_6$ wie eingangs definiert ist und $R_7$ ein Wasserstoffatom, eine Alkyl-, Phenylalkyl-, Phenyl-, Alkoxy-, $R_3R_4N-$, $R_3O-CO-$ oder $R_3R_4N-CO$-Gruppe darstellt, wobei $R_3$ und $R_4$ wie vorstehend erwähnt definiert sind,

ein vierter der Reste $X_1$ bis $X_5$ ein Sauerstoff-, Schwefel- oder Stickstoffatom oder eine $R_7C\leqslant$ Gruppe, in der $R_7$ wie vorstehend erwähnt definiert ist, oder auch eine Carbonylgruppe, wenn diese nicht zwischen zwei Stickstoffatomen steht,
ein fünfter der Reste $X_1$ bis $X_5$ ein Stickstoffatom, eine $R_7C\leqslant$ oder $(R_7)_2C<$ Gruppe, wobei $R_7$ wie vorstehend erwähnt definiert ist,
oder auch zwei benachbarte Reste der Reste $X_1$ bis $X_5$ zusammen eine o-Phenylengruppe bedeuten,
wobei, soweit nichts anderes erwähnt wurde,
die vorstehend erwähnten Alkyl-, Alkylen- oder Alkoxyteile jeweils 1 bis 3 Kohlenstoffatome enthalten können,
deren Tautomere, deren Stereoisomere einschließlich deren Gemische und deren Salze.

**2.** 5-gliedrige Heterocyclen der allgemeinen Formel I gemäß Anspruch 1, in der
einer der Reste $X_1$ bis $X_5$ eine Gruppe der Formeln
A - B - C - N< ,
A - B - C - CH< oder
A - B - C - $C\leqslant$ , in denen
A eine gegebenenfalls durch 1 bis 4 Alkylgruppen, durch eine Hydroxy-, Alkoxy-, Cyano-, Aminocarbonyl-, Carboxy- oder Alkoxycarbonylgruppe substituierte Cycloalkylgruppe mit 5 bis 7 Kohlenstoffatomen, in der eine unsubstituierte Methylengruppe durch die $R_a$-N< Gruppe ersetzt ist, wobei
$R_a$ ein Wasserstoffatom, eine Alkylgruppe, eine Phenylalkylgruppe, eine Alkoxycarbonylgruppe mit insgesamt 2 bis 6 Kohlenstoffatomen, eine Phenylalkoxycarbonylgruppe, eine Cycloalkoxycarbonylgruppe mit insgesamt 6 bis 8 Kohlenstoffatomen oder eine $R_1$-CO-O-($R_2$CH)-O-CO-Gruppe, in der
$R_1$ eine Alkylgruppe, eine Cycloalkylgruppe mit 5 bis 7 Kohlenstoffatomen, eine Alkoxygruppe mit 1 bis 5 Kohlenstoffatomen, eine Cycloalkoxygruppe mit 5 bis 7 Kohlenstoffatomen oder eine Phenylgruppe und
$R_2$ ein Wasserstoffatom, eine Alkylgruppe mit 1 bis 4 Kohlenstoffatomen darstellen,
und zusätzlich in den so gebildeten 6- oder 7-gliedrigen Azacycloalkylgruppen eine >CH- Einheit in 4-Stellung durch ein Stickstoffatom oder in den so gebildeten 5- bis 7-gliedrigen Azacycloalkylgruppen eine $-CH_2$-CH< Einheit durch eine -CH=C< Einheit ersetzt sein kann,
eine Pyridyl- oder Chinuclidinylgruppe oder auch, wenn
D eine durch eine $R_3R_4$N-CO-$NR_5$- oder $R_6$-$SO_2$-$NR_3$-Gruppe substituierte Alkylengruppe mit 1 bis 5 Kohlenstoffatomen, in denen
$R_3$ bis $R_5$, die gleich oder verschieden sein können, jeweils ein Wasserstoffatom, eine Alkyl- oder Phenylalkylgruppe und
$R_6$ eine Alkylgruppe mit 1 bis 5 Kohlenstoffatomen, eine Phenylalkyl- oder Phenylgruppe darstellen,
oder E eine durch eine $R_3R_4$N-, $R_6$CO-$NR_3$-, $R_6SO_2$-$NR_3$-, $R_3R_4$N-CO-$NR_5$-, Hydroxy- oder Alkoxygruppe substituierte geradkettige oder verzweigte Alkylengruppe mit jeweils 1 bis 5 Kohlenstoffatomen, in denen $R_3$ bis $R_6$ wie vorstehend erwähnt definiert sind, darstellen,
eine Phenylgruppe, die in 4-Stellung durch eine $R_a$NH-$CH_2$- oder $R_a$NH-C(=NH)-Gruppe substituiert ist, oder eine Phenylgruppe, an die über die Positionen 3 und 4 eine n-Propylen- oder n-Butylenbrücke angefügt ist, wobei die n-Propylen- und n-Butylenbrücke durch eine $R_a$NH-Gruppe substituiert ist und $R_a$ jeweils wie vorstehend erwähnt definiert ist,
B eine Bindung, eine $-CH_2-CH_2-$, $-OCH_2-$, $-CH_2O-$, -CON$R_3$- oder -$R_3$NCO-Gruppe, wobei $R_3$ wie vorstehend erwähnt definiert ist und ein Sauerstoff- oder Stickstoffatom des Restes B nicht direkt mit einem Stickstoffatom des Restes A oder des 5-gliedrigen Heterocyclus verbunden ist,
C eine Phenylengruppe, die durch Fluor-, Chlor- oder Bromatome, durch Alkyl-, Trifluormethyl-, Hydroxy- oder Alkoxygruppen mono- oder disubstituiert sein kann, wobei die Substituenten gleich oder verschieden sein können,
eine Pyridinylen-, Pyrimidinylen-, Pyrazinylen- oder Pyridazinylengruppe, die jeweils im Kohlenstoffgerüst durch eine Alkyl- oder Alkoxygruppe substituiert sein können,
eine 1,4-Cyclohexylen-, 1,4-Piperidinylen- oder 1,4-Piperazinylengruppe oder
auch, sofern nicht A eine Pyridylgruppe und gleichzeitig B eine Ethylen- oder CONH-Gruppe darstellt, eine Bindung, wobei gleichzeitig B nur dann eine Bindung sein kann, wenn A eine Amidinophenylgruppe darstellt,
ein zweiter der Reste $X_1$ bis $X_5$ eine Gruppe der Formeln
$R_b$O-CO - E - D - N< ,
$R_b$O-CO - E - D - CH< oder

$R_bO$-CO - E - D - C$\leqslant$ , in denen

D eine -CO-$NR_3$- oder -$NR_3$-CO-Gruppe, eine gegebenenfalls durch eine Hydroxy-, Alkoxy-, $R_3R_4N$-, $R_6CO$-$NR_3$-, $R_6O$-CO-$NR_3$-,$R_6SO_2$-$NR_3$- oder $R_3R_4N$-CO-$NR_5$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann sowie $R_3$ bis $R_6$ wie vorstehend erwähnt definiert sind,

eine Phenylengruppe, die durch Fluor-, Chlor- oder Bromatome, durch Alkyl-, Trifluormethyl-, Hydroxy- oder Alkoxygruppen mono- oder disubstituiert sein kann,

eine Pyridinylen-, Pyrimidinylen-, Pyrazinylen- oder Pyridazinylengruppe, die jeweils im Kohlenstoffgerüst durch eine Alkyl- oder Alkoxygruppe substituiert sein kann,

eine Cyclohexylengruppe, in der eine oder zwei >CH-Einheiten je durch ein Stickstoffatom ersetzt sein können, wobei zusätzlich eine zu einem Stickstoffatom benachbarte Methylengruppe durch eine Carbonylgruppe ersetzt sein kann, oder

eine über den Rest $W_1$ mit dem im Ring befindlichen Atom des jeweiligen Restes $X_1$ bis $X_5$ verknüpfte Alkylengruppe mit 1 bis 4 Kohlenstoffatomen, in der $W_1$ eine $NR_3$-Gruppe, in der $R_3$ wie vorstehend erwähnt definiert ist, ein Sauerstoff- oder Schwefelatom darstellt, wobei ein Sauerstoff- oder Schwefelatom des Restes $W_1$ nicht direkt an ein Stickstoffatom des 5-gliedrigen Heterocyclus gebunden sein kann,

E eine Bindung, wobei nicht gleichzeitig C eine Bindung und A eine 1-Methyl- oder 1-Benzyl-4-piperazinylgruppe darstellen kann, oder

eine gegebenenfalls durch eine Hydroxy-, Alkoxy-, $R_3R_4N$-, $R_6CO$-$NR_3$-, $R_6O$-CO-$NR_3$-, $R_6SO_2$-$NR_3$- oder $R_3R_4N$-CO-$NR_5$-Gruppe substituierte geradkettige oder verzweigte Alkylengruppe mit 1 bis 5 Kohlenstoffatomen, wobei $R_3$ bis $R_6$ wie vorstehend erwähnt definiert sind, oder

eine über den Rest $W_2$ mit dem Rest D verknüpfte Alkylengruppe, in der $W_2$ ein Sauerstoff- oder Schwefelatom, eine -$NR_3$-, -($R_6CO$)N- oder -($R_6SO_2$)N-Gruppe darstellt, in denen $R_3$ und $R_6$ wie vorstehend definiert sind und wobei ein Sauerstoff- oder Schwefelatom des Restes $W_2$ nicht direkt an ein Stickstoffatom des Restes D gebunden ist, und

$R_b$ ein Wasserstoffatom, eine Alkylgruppe mit 1 bis 5 Kohlenstoffatomen, eine Phenylalkylgruppe mit 1 bis 3 Kohlenstoffatomen im Alkylteil, eine Cycloalkylgruppe mit 5 bis 7 Kohlenstoffatomen oder eine $R_1$-CO-O-($R_2$CH)-Gruppe, in der $R_1$ und $R_2$ wie vorstehend erwähnt definiert sind, wobei der Abstand zwischen dem am weitesten entfernten Stickstoffatom der Gruppe A und der $COOR_b$-Gruppe mindestens 11 Bindungen beträgt,

ein dritter der Reste $X_1$ bis $X_5$ eine HN<, $R_6N$<, $R_7C\leqslant$ oder $(R_7)_2C$< Gruppe oder ein N-Atom, wobei $R_6$ wie eingangs definiert ist und $R_7$ ein Wasserstoffatom, eine Alkyl- oder Phenylgruppe darstellt,

ein vierter der Reste $X_1$ bis $X_5$ ein Sauerstoff-, Schwefel- oder Stickstoffatom oder eine $R_7C\leqslant$ Gruppe, in der $R_7$ wie vorstehend erwähnt definiert ist,

ein fünfter der Reste $X_1$ bis $X_5$ ein Stickstoffatom, eine $R_7C\leqslant$ oder $(R_7)_2C$< Gruppe, wobei $R_7$ wie vorstehend erwähnt definiert ist,

oder auch zwei benachbarte Reste der Reste $X_1$ bis $X_5$ zusammen eine o-Phenylengruppe bedeuten,

deren Tautomere, deren Stereoisomere einschließlich deren Gemische und deren Salze.

**3.** 5-gliedrige Heterocyclen der allgemeinen Formel I gemäß Anspruch 1, in der

einer der Reste $X_1$ bis $X_5$ eine Gruppe der Formeln

A - B - C - N< oder

A - B - C - C$\leqslant$ , in denen

A eine gegebenenfalls im Kohlenstoffgerüst durch 1 bis 4 Methylgruppen, durch eine Hydroxy-, Methoxy-, Cyano- oder Aminocarbonylgruppe substituierte 1,3-Pyrrolidinyl-, 1,3-Piperidyl- oder 1,4-Piperidylgruppe, wobei die vorstehend erwähnten Pyrrolidinyl- und Piperidylgruppen in 1-Stellung durch den Rest $R_a$ substituiert sind und

$R_a$ ein Wasserstoffatom, eine Methylgruppe, eine Ethylgruppe, eine Benzylgruppe oder eine Alkoxycarbonylgruppe mit insgesamt 2 bis 6 Kohlenstoffatomen darstellt,

eine 1,4-Piperazinyl- oder 3,4-Dehydro-1,4-piperidylgruppe, die jeweils in 1-Stellung durch den Rest $R_a$ substituiert sind und $R_a$ wie vorstehend erwähnt definiert ist,

eine Pyridyl- oder Chinuclidinylgruppe oder auch, wenn

D eine durch eine $R_6$-$SO_2$-$NR_3$-Gruppe substituierte Ethylengruppe, in der

$R_3$ ein Wasserstoffatom, eine Methyl- oder Ethylgruppe und

$R_6$ eine Alkylgruppe mit 1 bis 5 Kohlenstoffatomen oder eine Phenylgruppe darstellen,

oder E eine durch eine Amino-, $R_6CO$-$NR_3$-, $R_6SO_2$-$NR_3$- oder Hydroxygruppe substituierte

Ethylengruppe, in denen $R_3$ und $R_6$ wie vorstehend erwähnt definiert sind, darstellen,
eine Phenylgruppe, die in 4-Stellung durch eine $R_aNH-C(=NH)$-Gruppe substituiert ist,
B eine Bindung, eine $-CH_2-CH_2-$, $-OCH_2-$, $-CH_2O-$, $-CONR_3-$ oder $-R_3NCO$-Gruppe, wobei $R_3$ wie vorstehend erwähnt definiert ist und ein Sauerstoff- oder Stickstoffatom des Restes B nicht direkt mit einem Stickstoffatom des Restes A oder des 5-gliedrigen Heterocyclus verbunden ist,
C eine Phenylengruppe, die durch ein Chloratom oder durch eine Methylgruppe substituiert sein kann, eine Pyridinylen-, Pyrimidinylen-, Pyridazinylen- oder 1,4-Piperidinylengruppe
oder auch, sofern nicht A eine Pyridylgruppe und gleichzeitig B eine Ethylen- oder CONH-Gruppe darstellt, eine Bindung, wobei gleichzeitig B nur dann eine Bindung sein kann, wenn A eine Amidinophenylgruppe darstellt,
ein zweiter der Reste $X_1$ bis $X_5$ eine Gruppe der Formeln
$R_bO-CO - E - D - N<$ oder
$R_bO-CO - E - D - C\lessdot$ , in denen
D eine $-CO-NR_3-$ oder $-NR_3-CO$-Gruppe, wobei $R_3$ wie vorstehend erwähnt definiert ist, oder eine geradkettige oder verzweigte Alkylengruppe mit 1 bis 5 Kohlenstoffatomen,
eine durch eine $R_6SO_2-NR_3$-Gruppe substituierte Ethylengruppe,
eine Phenylengruppe, die durch ein Chloratom oder eine Methylgruppe substituiert sein kann,
eine 1,4-Cyclohexylengruppe oder
eine über eine $-NR_3$-Gruppe, wobei $R_3$ wie vorstehend erwähnt definiert ist, mit dem im Ring befindlichen Atom des jeweiligen Restes $X_1$ bis $X_5$ verknüpfte Alkylengruppe mit 1 bis 4 Kohlenstoffatomen,
E eine Bindung, wobei nicht gleichzeitig C eine Bindung und A eine 1-Methyl- oder 1-Benzyl-4-piperazinylgruppe darstellen kann, oder
eine gegebenenfalls durch eine $R_6CO-NR_3-$ oder $R_6SO_2-NR_3$-Gruppe substituierte Ethylengruppe, wobei $R_3$ und $R_6$ wie vorstehend erwähnt definiert sind, oder
eine $-O-CH_2-$ oder $-NR_3-CH_2$-Gruppe, wobei $R_3$ wie vorstehend erwähnt definiert ist, darstellt, und
$R_b$ ein Wasserstoffatom, eine Alkylgruppe mit 1 bis 5 Kohlenstoffatomen oder eine Cycloalkylgruppe mit 5 oder 6 Kohlenstoffatomen, wobei der Abstand zwischen dem am weitesten entfernten Stickstoffatom der Gruppe A und der $COOR_b$-Gruppe mindestens 11 Bindungen beträgt,
ein dritter der Reste $X_1$ bis $X_5$ eine $HN<$, $R_6N<$ oder $R_7C\lessdot$ Gruppe oder ein N-Atom, wobei $R_6$ wie vorstehend erwähnt definiert ist und $R_7$ ein Wasserstoffatom, eine Methyl- oder Ethylgruppe darstellen,
ein vierter der Reste $X_1$ bis $X_5$ ein Sauerstoff-, Schwefel- oder Stickstoffatom oder eine $R_7C\lessdot$ Gruppe, in der $R_7$ wie vorstehend erwähnt definiert ist,
ein fünfter der Reste $X_1$ bis $X_5$ ein Stickstoffatom oder eine $R_7C\lessdot$ Gruppe, wobei $R_7$ wie vorstehend erwähnt definiert ist,
oder auch zwei benachbarte Reste der Reste $X_1$ bis $X_5$ zusammen eine o-Phenylengruppe bedeuten,
deren Tautomere, deren Stereoisomere einschließlich deren Gemische und deren Salze.

**4.** 5-gliedrige Heterocyclen der allgemeinen Formel I gemäß Anspruch 1, in der
einer der Reste $X_1$ bis $X_5$ eine Gruppe der Formeln
A - B - C - N< oder
$A - B - C - C\lessdot$ , in denen
A eine in 1-Stellung durch den Rest $R_a$ substituierte 4-Piperidylgruppe, wobei
$R_a$ ein Wasserstoffatom, eine Benzylgruppe oder eine Alkoxycarbonylgruppe mit insgesamt 2 bis 6 Kohlenstoffatomen darstellt,
eine 1,4-Piperazinylgruppe, die in 1-Stellung durch den Rest $R_a$ substituiert ist, wobei $R_a$ wie vorstehend erwähnt definiert ist
oder auch, wenn
D eine durch eine $R_6-SO_2-NR_3$-Gruppe substituierte Ethylengruppe, in der
$R_3$ ein Wasserstoffatom und
$R_6$ eine Alkylgruppe mit 1 bis 5 Kohlenstoffatomen darstellen,
eine Phenylgruppe, die in 4-Stellung durch eine $NH_2-C(=NH)$-Gruppe substituiert ist,
B eine Bindung, eine $-CH_2-CH_2-$ oder $-CH_2O$-Gruppe,
C eine Phenylen- oder eine 1,4-Piperidinylengruppe oder, falls B nicht gleichzeitig ebenfalls eine Bindung darstellt, auch eine Bindung,
ein zweiter der Reste $X_1$ bis $X_5$ eine Gruppe der Formel
$R_bO-CO - E - D - C\lessdot$ , in der
D eine -CO-NH-Gruppe, eine Alkylengruppe mit 1 bis 5 Kohlenstoffatomen, eine Phenylengruppe, eine

1,4-Cyclohexylengruppe oder eine durch eine $R_6$-$SO_2$-$NR_3$-Gruppe substituierte Ethylengruppe,
E eine Bindung, wobei nicht gleichzeitig C eine Bindung und A eine 1-Benzyl-4-piperazinylgruppe darstellen kann, oder eine Ethylengruppe und
$R_b$ ein Wasserstoffatom oder eine Alkylgruppe mit 1 bis 4 Kohlenstoffatomen, wobei der Abstand zwischen dem am weitesten entfernten Stickstoffatom der Gruppe A und der $COOR_b$-Gruppe mindestens 11 Bindungen beträgt,
ein dritter der Reste $X_1$ bis $X_5$ eine Phenyl-N< oder $R_7C$≪ Gruppe oder ein N-Atom, wobei $R_7$ ein Wasserstoffatom, eine Methyl- oder Ethylgruppe darstellt,
ein vierter der Reste $X_1$ bis $X_5$ ein Sauerstoff-, Schwefel- oder Stickstoffatom oder eine $R_7C$≪ Gruppe, in der $R_7$ wie vorstehend erwähnt definiert ist,
ein fünfter der Reste $X_1$ bis $X_5$ ein Stickstoffatom bedeuten,
deren Tautomere, deren Stereoisomere einschließlich deren Gemische und deren Salze.

5. Folgende Verbindungen der allgemeinen Formel I gemäß Anspruch 1:
   (i) 2-(trans-4-Carboxy-cyclohexyl)-5-[4-(4-piperidyl)-phenyl]-1,3,4-thiadiazol,
   (ii) 2-[trans-4-(Methoxycarbonyl)-cyclohexyl]-5-[4-(4-piperidyl)-phenyl]-1,3,4-thiadiazol,
   (iii) 1-[6-(4-Amidino-phenyl)-3-pyridazinyl]-4-[2-(n-butansulfonylamino)-2-carboxy-ethyl]-imidazol,
   (iv) 1-[6-(4-Amidino-phenyl)-3-pyridazinyl]-4-[2-(n-butansulfonylamino)-2-(methoxycarbonyl)-ethyl]-imidazol,
   (v) 2-[trans-4-(Isobutyloxycarbonyl)-cyclohexyl]-5-[4-(4-piperidinyl)-phenyl]-1,3,4-thiadiazol und
   (vi) 2-[trans-4-(Ethyloxycarbonyl)-cyclohexyl]-5-[4-(4-piperidinyl)-phenyl]-1,3,4-thiadiazol
   sowie deren Salze.

6. Physiologisch verträgliche Additionssalze der Verbindungen nach mindestens einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Additionssalz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

9. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der 5-gliedrigen Heterocyclen gemäß den Ansprüchen 1 bis 7, dadurch gekennzeichnet, daß
   a) zur Herstellung von Verbindungen der allgemeinen Formel I, in der ein zweiter der Reste $X_1$ bis $X_5$ eine $R_bO$-CO-E-D-CH<- oder $R_bO$-CO-E-D-C≪ Gruppe darstellt, eine Verbindung der allgemeinen Formel

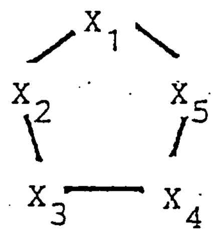

, (II)

   in der
   $X_1$ bis $X_5$ mit der Maßgabe wie in den Ansprüchen 1 bis 5 definiert sind, daß ein zweiter der Reste $X_1$ bis $X_5$ eine HO-CO-CH< -oder HO-CO-C≪ Gruppe darstellt, oder deren reaktionsfähigen Derivate mit einer Verbindung der allgemeinen Formel

$R_bO$ - CO - E - $HNR_3$ ,(III)

in der
$R_3$, $R_b$ und E wie in den Ansprüchen 1 bis 5 definiert sind, umgesetzt wird oder
b) zur Herstellung von Verbindungen der allgemeinen Formel I, in der $R_a$ ein Wasserstoffatom darstellt, ein Schutzrest von einer Verbindung der allgemeinen Formel

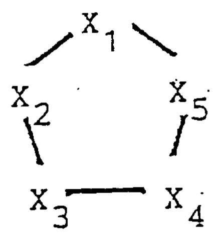

, (IV)

in der
$X_1$ bis $X_5$ mit der Maßgabe wie in den Ansprüchen 1 bis 5 definiert sind, daß $R_a$ eine Alkoxycarbonylgruppe mit insgesamt 2 bis 6 Kohlenstoffatomen, eine Phenylalkoxycarbonylgruppe, eine Alkenyloxycarbonylgruppe mit insgesamt 4 bis 6 Kohlenstoffatomen, eine Cycloalkoxycarbonylgruppe mit insgesamt 6 bis 8 Kohlenstoffatomen oder einen abspaltbaren Schutzrest für eine Iminogruppe darstellt, mittels Hydrolyse, Hydrogenolyse oder Thermolyse abgespalten wird oder
c) zur Herstellung von Verbindungen der allgemeinen Formel I, in der $R_b$ ein Wasserstoffatom darstellt, ein Schutzrest von einer Verbindung der allgemeinen Formel

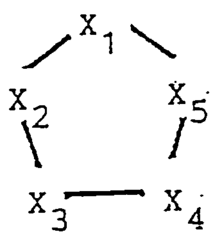

, (V)

in der
$X_1$ bis $X_5$ mit der Maßgabe wie in den Ansprüchen 1 bis 5 definiert sind, daß $R_b$ eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil oder einen abspaltbaren Schutzrest für eine Carboxygruppe darstellt, mittels Hydrolyse, Hydrogenolyse oder Thermolyse abgespalten wird oder
d) zur Herstellung von 1,3,4-Oxadiazol-, 1,2,4-Triazol- und 1,3,4-Thiadiazolderivaten der allgemeinen Formel I, eine gegebenenfalls im Reaktionsgemisch gebildete Verbindung der allgemeinen Formel

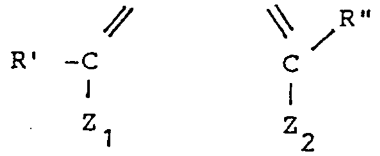

, (VI)

in der
$Z_1$ und $Z_2$, die gleich oder verschieden sein können, Halogenatome, gegebenenfalls durch $R_6$ substituierte Aminogruppen, Hydroxy-, Alkoxy-, Mercapto- oder Alkylmercaptogruppen, einer der Reste R' oder R" eine A-B-C-Gruppe und der andere der Reste R' oder R" eine $R_bO$-CO-E-D-Gruppe darstellen, cyclisiert und erforderlichenfalls eine so erhaltene Verbindung alkyliert wird oder

e) zur Herstellung von Verbindungen der allgemeinen Formel I, in der A eine Phenylgruppe darstellt, die in 4-Stellung durch eine $R_aNH-C(=NH)$-Gruppe substituiert ist, eine gegebenenfalls im Reaktionsgemisch gebildete Verbindung der allgemeinen Formel

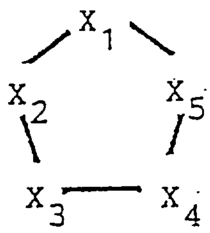

, (VII)

in der
$X_1$ bis $X_5$ mit der Maßgabe wie in den Ansprüchen 1 bis 5 definiert sind, daß A eine Phenylgruppe darstellt, die in 4-Stellung durch eine $Z_3-C(=NH)$-Gruppe substituiert ist, wobei $Z_3$ eine Alkoxy-, Aralkoxy-, Alkylthio-, Aralkylthio- oder Aminogruppe darstellt, mit einem Amin der allgemeinen Formel

$R_a' - NH_2$ ,(VIII)

in der
$R_a'$ ein Wasserstoffatom oder eine Alkylgruppe mit 1 bis 3 Kohlenstoffatomen darstellt, umgesetzt wird oder
f) zur Herstellung von Verbindungen der allgemeinen Formel I, in der A eine Phenylgruppe darstellt, die in 4-Stellung durch eine $R_aNH-C(=NH)$-Gruppe substituiert ist, eine Verbindung der allgemeinen Formel

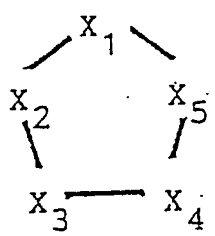

, (IX)

in der
$X_1$ bis $X_5$ mit der Maßgabe wie in den Ansprüchen 1 bis 5 definiert sind, daß A eine Phenylgruppe darstellt, die in 4-Stellung durch eine Cyanogruppe substituiert ist, mit Hydroxylamin umgesetzt und anschließend ein so erhaltenes Amidoxim reduziert wird oder
g) zur Herstellung von Verbindungen der allgemeinen Formel I, in der A eine Phenylgruppe darstellt, die in 4-stellung durch eine $R_aNH-C(=NH)$-Gruppe substituiert ist, eine Verbindung der allgemeinen Formel

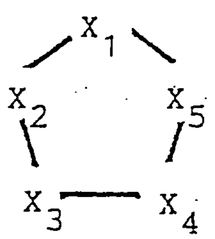

, (IX)

in der
$X_1$ bis $X_5$ mit der Maßgabe wie in den Ansprüchen 1 bis 5 definiert sind, daß A eine Phenylgruppe darstellt, die in 4-Stellung durch eine Cyanogruppe substituiert ist, mit einem entsprechenden

Alkylchloroaluminiumamid umgesetzt wird oder
h) zur Herstellung von 1,3-Thiazolen und Imidazolen der allgemeinen Formel I, eine Verbindung der allgemeinen Formel

$R' - CO - CH_2 - Z_4$ ,(X)

mit einer Verbindung der allgemeinen Formel

$R'' - CU - NH_2$ ,(XI)

in denen
einer der Reste R' oder R'' eine A-B-C-Gruppe und der andere der Reste R' oder R'' eine $R_bO$-CO-E-D-Gruppe, $Z_4$ eine nukleophile Austrittsgruppe und
U ein Schwefelatom oder eine Iminogruppe darstellen, umgesetzt wird oder
i) zur Herstellung von Verbindungen der allgemeinen Formel I, in der $R_a$ mit Ausnahme des Wasserstoffatoms wie eingangs definiert ist und B eine $-CH_2O$-Gruppe darstellt, eine Verbindung der allgemeinen Formel

$A - CH_2 - Z_5$ ,(XII)

in der
A mit der Maßgabe wie in den Ansprüchen 1 bis 5 definiert ist, daß $R_a$ mit Ausnahme des Wasserstoffatoms wie in den Ansprüchen 1 bis 5 definiert ist, und
$Z_5$ eine nukleophile Austrittsgruppe darstellt, mit einer Verbindung der allgemeinen Formel

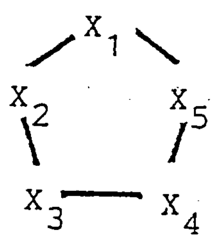

, (XIII)

in der
$X_1$ bis $X_5$ mit der Maßgabe wie in den Ansprüchen 1 bis 5 definiert sind, daß einer der Reste $X_1$ bis $X_5$ eine HO-C-CH<- oder HO-C-C< Gruppe darstellt, wobei C wie in den Ansprüchen 1 bis 5 definiert ist, oder mit deren Alkali- oder Erdalkalimetallsalzen umgesetzt wird oder
j) zur Herstellung von Verbindungen der allgemeinen Formel I, in der einer der Reste $X_1$ bis $X_5$ eine A-B-C-N< oder $R_bO$-CO-E-D-N< Gruppe darstellt, eine Verbindung der allgemeinen Formel

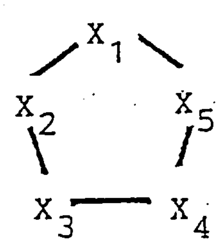

, (XIV)

in der
$X_1$ bis $X_5$ mit der Maßgabe wie in den Ansprüchen 1 bis 5 definiert sind, daß einer der Reste $X_1$ bis $X_5$ eine Iminogruppe darstellt, mit einer Verbindung der allgemeinen Formel

$W - Z_6$ ,(XV)

in der

W eine A-B-C- oder $R_bO$-CO-E-D-Gruppe, wobei A bis D wie in den Ansprüchen 1 bis 5 erwähnt definiert sind, und

$Z_6$ eine nukleophile Austrittsgruppe darstellen, alkyliert wird oder

k) zur Herstellung von Verbindungen der allgemeinen Formel I, in der $R_b$ eine $R_1$-CO-O-($R_2$CH)-Gruppe, in der $R_1$ und $R_2$ wie in den Ansprüchen 1 bis 5 definiert sind, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil darstellt, eine Verbindung der allgemeinen Formel

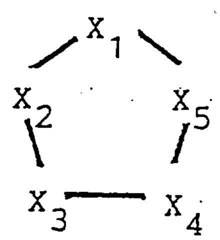

, (XVI)

in der

$X_1$ bis $X_5$ mit der Maßgabe wie in den Ansprüchen 1 bis 5 definiert sind, daß $R_b$ ein Wasserstoffatom darstellt, mit einer Verbindung der allgemeinen Formel

$Z_7$ - $R_b'$ ,(XVII)

in der

$R_b'$ eine $R_1$-CO-O-($R_2$CH)-Gruppe, in der $R_1$ und $R_2$ wie in den Ansprüchen 1 bis 5 definiert sind, eine Alkylgruppe mit 1 bis 5 Kohlenstoffatomen, eine Alkenylgruppe mit 3 bis 5 Kohlenstoffatomen, eine Phenylalkylgruppe mit 1 bis 3 Kohlenstoffatomen im Alkylteil, eine Cycloalkyl- oder Cycloalkylalkylgruppe mit jeweils 5 bis 7 Kohlenstoffatomen im Cycloalkylteil darstellt und

$Z_7$ eine Hydroxygruppe oder eine nukleophile Austrittsgruppe darstellen, verestert wird und

erforderlichenfalls ein während den Umsetzungen zum Schutze von reaktiven Gruppen verwendeter Schutzrest abgespalten wird und/oder

gewünschtenfalls eine so erhaltene Verbindung der allgemeinen Formel I in ihre Stereoisomere aufgetrennt wird und/oder eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 94 10 1125

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.5) |
|---|---|---|---|
| P,X | EP-A-0 525 629 (THOMAE) 3. Februar 1993<br>* das ganze Dokument *<br>* RN 149868-60-0; 2-Thiazolepropanoic acid, 4-[4-[1-(aminoiminomethyl)-4-piperidinyl]phenyl]-, methyl ester *<br>* RN 149868-44-0; 2-Thiazolepropanoic acid, 4-[4-(4-piperidinyl)phenyl]-, methyl ester *<br>--- | 1-10 | C07D417/10<br>C07D403/04<br>A61K31/445<br>A61K31/41<br>C07D401/10<br>C07D417/06<br>C07D401/06<br>C07D403/12<br>C07D417/12<br>C07D417/14<br>C07D417/04<br>C07D413/10 |
| A | WO-A-92 01672 (NOVO NORDISK) 6. Februar 1992<br>* RN 141360-19-2; 2,5-Pyrrolidinedione, 1-[4-[1-butyl-3-[[4- (trifluoromethyl)phenoxy]methyl]-4-piperidinyl]phenyl]-, monohydrochloride, trans-(.+-.)- *<br>---<br>-/-- | 1-10 | |

RECHERCHIERTE SACHGEBIETE (Int.Cl.5)

C07D

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 25. April 1994 | Kissler, B |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.........................................................
& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 94 10 1125

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| X | JOURNAL OF MEDICINAL CHEMISTRY.<br>Bd. 19, Nr. 11 , 1976 , WASHINGTON US<br>Seiten 1315 - 1324<br>Bauer, Victor J.; Duffy, Brian J.; Hoffman, David; Klioze, Solomon S.; Kosley, Raymond W., Jr.; McFadden, Arthur R.; Martin, Lawre 'Synthesis of spiro[isobenzofuran-1(3H),4'-piperidines] as potential central nervous system agents. 1'<br>* RN 60081-44-9; 4-Piperidinol, 4-[2-(4,5-dihydro-4,4-dimethyl-2-oxazolyl)-4-methoxyphenyl]-1-methyl- *<br>----- | 1-3 | |
| | | | RECHERCHIERTE SACHGEBIETE (Int.Cl.5) |

EPO FORM 1503 03.82 (P04C12)

EP 94 10 1125
-C-

Mangelnde Genauigkeit

Die Definition des/der folgenden Substituenten ist zu allgemein und/oder umfasst einen zu grossen Bereich von chemisch grundverschiedenen Resten und ist nur teilweise durch Beispiele in der Beschreibung gestützt:

X1, X2, X3, X4, X5, A, B, C

Die grosse Zahl der sich aus der Kombination aller beanspruchten Substituenten der obige Liste ergebenden, theoretisch denkbaren Verbindungen schliesst eine umfassende Recherche aus. In Anlehnung an den Geist und das erfinderische Konzept der vorliegenden Anmeldung die Recherche auf den/die folgenden Fall/Fälle beschränkt worden:
5-gliedrige Heterocyclen (nicht weiter condensiert) mit mindestens einem N-Atom und mit einem 4-Piperidinylphenyl- (o,m oder p-subst.) Substituenten.

(Vgl. Art. 83, 84 EPC, Regel 45 EPC und Richtlinien zur Durchführung Teil B, Kapitel III .7).